# EUROPEAN PATENT SPECIFICATION

(11) **EP 3 613 334 B1**
(45) Date of publication and mention of the grant of the patent: **02.09.2026**
(21) Application number: 18805965.3
(22) Date of filing: 26.03.2018
(51) Int. Cl.: A61B 3/113, A61B 10/00, A61B 5/16, A61B 3/00

(54) **EVALUATING DEVICE, EVALUATING METHOD, AND EVALUATING PROGRAM**
EVALUIERUNGSGERÄT, EVALUIERUNGSVERFAHREN UND EVALUIERUNGSPROGRAMM
DISPOSITIF D'ÉVALUATION, PROCÉDÉ D'ÉVALUATION ET PROGRAMME D'ÉVALUATION

(30) Priority: 22.05.2017 JP 2017100871
(43) Date of publication of application: 26.02.2020
(73) Proprietor: JVCKENWOOD Corporation, Yokohama-shi, Kanagawa 221-0022 (JP)
(72) Inventor: SHUDO, Katsuyuki, Yokohama-shi Kanagawa 221-0022 (JP)
(74) Representative: Wimmer, Hubert
(86) International application number: PCT/JP2018/012230
(87) International publication number: WO 2018/216347

(56) References cited:
- EP-A1- 2 829 221
- WO-A1-2014/164453
- WO-A2-2016/040673
- JP-A- 2003 038 443
- JP-A- 2014 068 937
- JP-A- 2015 144 635
- JP-B2- 5 983 135

## Description

### Field

The present invention relates to an evaluation device, an evaluation method, and an evaluation program.

### Background

As an eye tracking technique, the corneal reflection method has been known. In the corneal reflection method, a subject is irradiated with infrared light emitted from a light source, and an eye ball of the subject irradiated with the infrared light is imaged with a camera, and a position of a pupil with respect to a corneal reflection image, which is a reflection image of the light source on a surface of the cornea, is detected, thereby detecting a line of sight of the subject.

By using a result from such detection of a line of sight of the subject, various kinds of evaluations are made. For example, JP-A-2003-038443 describes a technique of inspecting a brain function by detecting eye movement.

EP 2 829 221 A discloses an autism diagnosis support method and system, and autism diagnosis support device by using a conventionally proposed "eye-gaze detection technique". For example, a method for supporting autism diagnosis for a subject, uses an eye-gaze detecting unit at least including a camera portion capturing an image of an eye of the subject, or an electrode portion to be mounted on a head of the subject and detecting a movement of the eye, or a display portion to be disposed at a position in an eye gaze direction of the subject. The method includes: displaying, on a screen of the display portion, a combination image for sequentially displaying at least two images including a predetermined human image and a predetermined non-human image; and evaluating an eye-gaze position of the subject by detecting eye-gaze position information on the subject looking at the combination image in use of the eye-gaze detecting unit, then inputting the eye-gaze position information on the subject in an eye-gaze position information storing portion, and comparing, based on an eye-gaze position evaluation algorithm, the eye-gaze position information on the subject with eye-gaze position information on an individual with autism and/or a typically developing individual.

JP 5 983 135 B discloses a diagnosis support apparatus which includes: a display part; a display control part; a view point detecting part; and a determination part. The display part includes: a first display area partially intercepted by a plurality of intercepting parts; and second and third display areas not intercepted by the plurality of intercepting parts. The display control part allows the display part to display a first image on the first display area so that the first image passes through the slits between the plurality of intercepting parts, display the first image in one of the second display area and the third area after the first image passes through the slits, and display a second image different from the first image in the other. The view point detecting part detects a view point of a subject in the first display area, the second display area and the third display area. The determination part determines the degree of development disorder on the basis of the detection result for the view points.

WO 2016 040673 A discloses an animation based autism spectrum disorder assessment.

WO 2014 164453 A discloses a methods and system for assessing a human subject's neurological and/or psychological status. The methods entail displaying visual tests to a human subject, wherein each of the visual tests includes a visual target signal, optionally with visual cue signals, for eliciting visual and, optionally, body part, movements by the subject. Following the display, the movements are then detected. The latency and/or correctness of such movements can then be used to assess the subject's neurological and/or psychological status.

### Summary

### Technical Problem

It is said that people affected by cranial nerve disease and disorder, such as dementia, have been increasing in recent years. Moreover, there have been demands for early discovery of such cranial nerve disease and disorder and quantitative evaluation of the severity of the symptoms. For example, it has been known that symptoms of cranial nerve disease and disorder affect memory. Therefore, to evaluate a subject highly accurately by inspecting the memory of the subject has been demanded.

The present invention has been made in view of the above circumstances, and has an object to provide an evaluation device, an evaluation method, and an evaluation program that are capable of performing evaluation of a subject with high accuracy.

### Solution to Problem

In accordance with the present invention, an evaluation device, an evaluation method, and evaluation program as set forth in the appended claims is provided. An evaluation device according to the present invention includes the features of claim 1.

An evaluation method according to the present invention includes the features of claim 6.

An evaluation program according to the present invention is defined in claim 7.

### Advantageous Effects of Invention

According to the present invention, it is possible to perform evaluation with high accuracy by using a result of detection of a line of sight of a subject.

### Brief Description of Drawings

FIG. 1 is a schematic perspective view of an example of an eye tracking device according to a first embodiment.
FIG. 2 is a diagram schematically illustrating a positional relation among a display device, a stereo camera device, an illumination device, and an eyeball of a subject according to the embodiment.
FIG. 3 is a diagram illustrating an example of a hardware configuration of the eye tracking device according to the embodiment.
FIG. 4 is a functional block diagram illustrating an example of the eye tracking device according to the embodiment.
FIG. 5 is a schematic diagram for explaining a calculation method of position data of a corneal curvature center according to the embodiment.
FIG. 6 is a schematic diagram for explaining a calculation method of position data of a corneal curvature center according to the embodiment.
FIG. 7 is a flowchart showing an example of an eye tracking method according to the embodiment.
FIG. 8 is a schematic diagram for explaining an example of calibration process according to the embodiment.
FIG. 9 is a flowchart showing an example of calibration process according to the embodiment.
FIG. 10 is a schematic diagram for explaining an example of gaze-point detection process according to the embodiment.
FIG. 11 is a flowchart showing an example of the gaze-point detection process according to the embodiment.
FIG. 12 is a diagram illustrating an example of an image displayed on a display device by a display control unit according to the embodiment.
FIG. 13 is a diagram illustrating an example of movement of a gaze point of a subject.
FIG. 14 is a diagram illustrating an example of an image displayed on the display device by the display control unit according to the embodiment.
FIG. 15 is a diagram illustrating an example of an image displayed on the display device by the display control unit according to the embodiment.
FIG. 16 is a diagram illustrating an example of an image displayed on the display device by the display control unit according to the embodiment.
FIG. 17 is a diagram illustrating an example of an image displayed on the display device by the display control unit according to the embodiment.
FIG. 18 is a diagram illustrating an example of an image displayed on the display device by the display control unit according to the embodiment.
FIG. 19 is a diagram illustrating an example of an image displayed on the display device by the display control unit according to the embodiment.
FIG. 20 is a diagram illustrating an example of an image displayed on the display device by the display control unit according to the embodiment.
FIG. 21 is a time chart showing a time at which each image is displayed.
FIG. 22 is a flowchart showing an example of an evaluation method according to the embodiment.
FIG. 23 is a flowchart showing an example of an evaluation method according to a second embodiment. Description of Embodiments

Hereinafter, embodiments according to the present invention are described with reference to the drawings, but the present invention is not limited thereto.

In the following description, positional relations of respective portions are described, setting a three-dimensional global coordinate system. A direction parallel to a first axis of a predetermined plane is referred to as an X-axis direction, a direction parallel to a second axis of the predetermined plane perpendicular to the first axis is referred to as a Y-axis direction, and a direction parallel to a third axis that is perpendicular to both the first axis and the second axis is referred to as a Z-axis direction. The predetermined plane includes an XY plane.

### First Embodiment

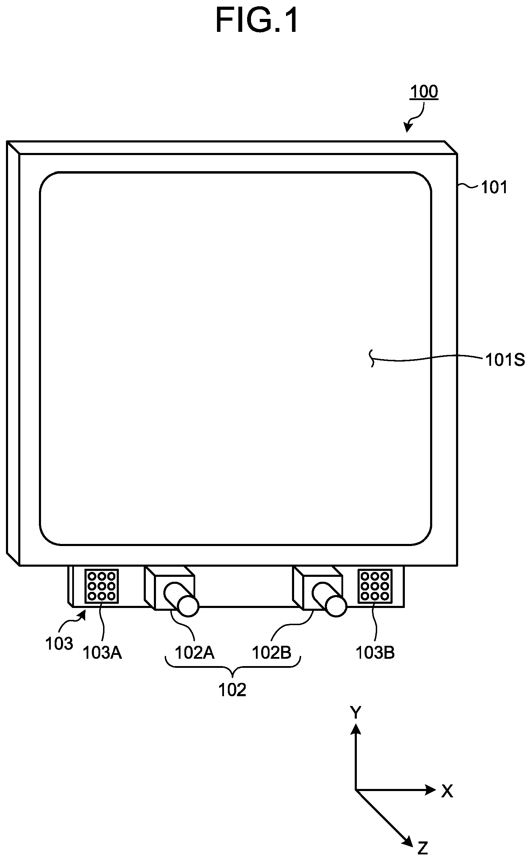

A first embodiment is described. FIG. 1 is a schematic perspective view of an example of an eye tracking device 100 according to the present embodiment. In the present embodiment, the eye tracking device 100 is used as an evaluation device to evaluate a target of interest of a subject.

As illustrated in FIG. 1, the eye tracking device 100 includes a display device 101, a stereo camera device 102, and an illumination device 103.

The display device 101 includes a flat panel display, such as a liquid crystal display (LCD) or an organic electroluminescence display (OLED). The display device 101 functions as a display unit.

In the present embodiment, a display screen 101S of the display device 101 is substantially parallel to the XY plane. The X-axis direction is a horizontal direction of the display screen 101S, and the Y-axis direction is a vertical direction of the display screen 101S, and the Z-axis direction is a depth direction perpendicular to the display screen 101S.

The stereo camera device 102 includes a first camera 102A and a second camera 102B. The stereo camera device 102 is arranged below the display screen 101S of the display device 101. The first camera 102A and the second camera 102B are arranged in the X-axis direction. The first camera 102A is arranged in the -X direction relative to the second camera 102B. Each of the first camera 102A and the second camera 102B includes an infrared camera, and includes an infrared ray camera, and has an optical system that allows near infrared light having, for example, a wavelength of 850 [nm] to pass through, and an imaging device that can receive the near infrared light.

The illumination device 103 includes a first light source 103A and a second light source 103B. The illumination device 103 is arranged below the display screen 101S of the display device 101. The first light source 103A and the second light source 103B are arranged in the X-axis direction. The first light source 103A is arranged in the -X direction relative to the first camera 102A. The second light source 103B is arranged in the +X direction relative to the second camera 102B. Each of the first light source 103A and the second light source 103B includes a light emitting diode (LED) light source, and is capable of emitting near infrared light having, for example, a wavelength of 850 [nm]. The first light source 103A and the second light source 103B may be arranged between the first camera 102A and the second camera 102B.

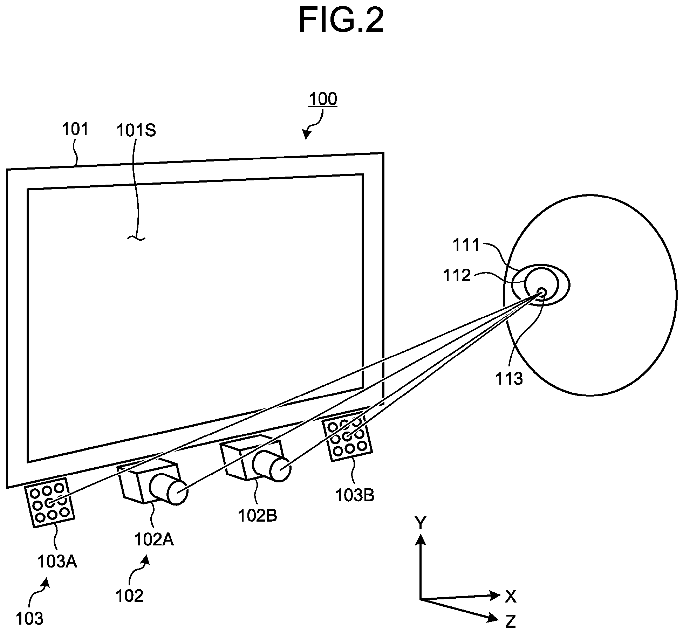

FIG. 2 is a diagram schematically illustrating a positional relation among the display device 101, the stereo camera device 102, the illumination device 103, and an eyeball 111 of a subject according to the present embodiment.

The illumination device 103 emits near infrared light, which is the detection light, to illuminate the eyeball 111 of the subject. The stereo camera device 102 images the eyeball 111 with the second camera 102B when the eyeball 111 is irradiated with the detection light emitted from the first light source 103A, and images the eyeball 111 with the first camera 102A when the eyeball 111 is irradiated with the detection light emitted from the second light source 103B.

From at least one of the first camera 102A and the second camera 102B, a frame synchronization signal is output. The first light source 103A and the second light source 103B emit the detection light based on the frame synchronization signal. The first camera 102A acquires image data of the eyeball 111 when the eyeball 111 is irradiated with the detection light emitted from the second light source 103B. The second camera 102B acquires image data of the eyeball 111 when the eyeball 111 is irradiated with the detection light emitted from the first light source 103A.

When the eyeball 111 is irradiated with the detection light, a part of the detection light is reflected on a pupil 112, and light from the pupil 112 enters the stereo camera device 102. Moreover, when the eyeball 111 is irradiated with the detection light, a corneal reflection image 113, which is a virtual image of a cornea, is formed on the eyeball 111, and light from the corneal reflection image 113 enters the stereo camera device 102.

As relative positions among the first camera 102A, the second camera 102B, the first light source 103A, and the second light source 103B are appropriately set, the intensity of light entering the stereo camera device 102 from the pupil 112 becomes low, and the intensity of light entering the stereo camera device 102 from the corneal reflection image 113 becomes high. That is, the image of the pupil 112 acquired by the stereo camera device 102 has low intensity, and the image of the corneal reflection image 113 has high intensity. The stereo camera device 102 can detect a position of the pupil 112 and the position of the corneal reflection image 113 based on the intensity of an acquired image.

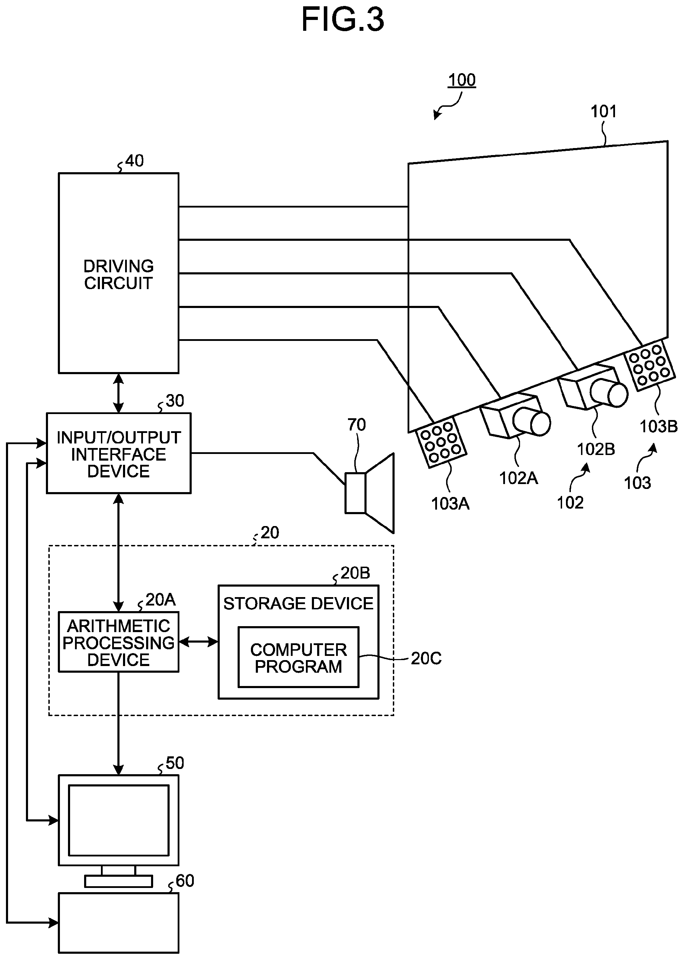

FIG. 3 is a diagram illustrating an example of a hardware configuration of the eye tracking device 100 according to the present embodiment. As illustrated in FIG. 3, the eye tracking device 100 includes the display device 101, the stereo camera device 102, the illumination device 103, a computer system 20, an input/output interface device 30, a driving circuit 40, an output device 50, an input device 60, and a voice output device 70. The computer system 20 includes an arithmetic processing device 20A and a storage device 20B.

The computer system 20, the driving circuit 40, the output device 50, the input device 60, and the voice output device 70 perform data communication through the input/output interface device 30.

The arithmetic processing device 20A includes a microprocessor, such as a central processing unit (CPU). The storage device 20B includes a memory or a storage, such as a read-only memory (ROM) and a random access memory (RAM). The arithmetic processing device 20A performs arithmetic processing according to a computer program 20C stored in the storage device 20B.

The driving circuit 40 generates a driving signal, and outputs it to the display device 101, the stereo camera device 102, and the illumination device 103. Moreover, the driving circuit 40 supplies image data of the eyeball 111 acquired by the stereo camera device 102 to the computer system 20 through the input/output interface device 30.

The output device 50 includes a display device, such as a flat panel display. The output device 50 may include a printer device. The input device 60 generates input data by being operated. The input device 60 includes a keyboard or a mouse for a computer system. The input device 60 may include a touch sensor that is arranged on a display screen of the output device 50. The voice output device 70 includes a speaker, and outputs voice, for example, to call attention from the subject.

In the present embodiment, the display device 101 and the computer system 20 are separate devices. The display device 101 and the computer system 20 may be unified. For example, when the eye tracking device 100 includes a tablet personal computer, the tablet personal computer may be equipped with the computer system 20, the input/output interface device 30, the driving circuit 40, and the display device 101.

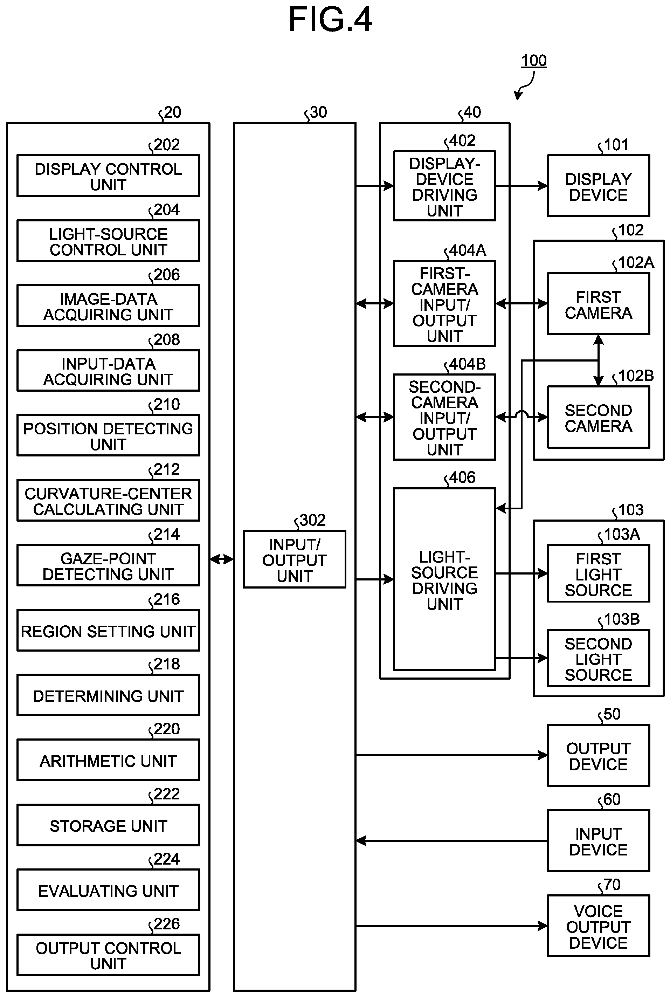

FIG. 4 is a functional block diagram illustrating an example of the eye tracking device 100 according to the present embodiment. As illustrated in FIG. 4, the input/output interface device 30 includes an input/output unit 302. The driving circuit 40 includes a display-device driving unit 402 that generates a driving signal to drive the display device 101, and outputs it to the display device 101, a first-camera input/output unit 404A that generates a driving signal to drive the first camera 102A, and outputs it to the first camera 102A, a second-camera input/output unit 404B that generates a driving signal to drive the second camera 102B, and outputs it to the second camera 102B, and a light-source driving unit 406 that generates a driving signal to drive the first light source 103A and the second light source 103B, and outputs it to the first light source 103A and the second light source 103B. Moreover, the first-camera input/output unit 404A supplies image data of the eyeball 111 that is acquired by the first camera 102A to the computer system 20 through the input/output unit 302. The second-camera input/output unit 404B supplies image data of the eyeball 111 that is acquired by the second camera 102B to the computer system 20 through the input/output unit 302.

The computer system 20 controls the eye tracking device 100. The computer system 20 includes a display control unit 202, a light-source control unit 204, an image-data acquiring unit 206, an input-data acquiring unit 208, a position detecting unit 210, a curvature-center calculating unit 212, a gaze-point detecting unit 214, a region setting unit 216, a determining unit 218, an arithmetic unit 220, a storage unit 222, an evaluating unit 224, and an output control unit 226. Functions of the computer system 20 are implemented by the arithmetic processing device 20A and the storage device 20B.

The display control unit 202 repeats a display operation to display plural objects on the display screen 101S and a non-display operation to hide the objects in predetermined timing after the display operation is started. A period in which plural objects are displayed by the display operation is referred to as a display period, and a period in which plural objects are hidden by the non-display operation is referred to as non-display period. The display control unit 202 displays an image to be shown to the subject on the display screen 101S of the display device 101. This image includes a scene in which plural objects are shown and a scene in which the plural objects are hidden. Therefore, the display control unit 202 is configured to perform the display operation in which plural objects are displayed on the display screen 101S and the non-display operation in which the plural objects are hidden. This image includes a scene in which a range region indicating a range of a corresponding region that corresponds to the plural objects is displayed. Moreover, this image includes a scene in which character information to give an instruction to the subject and the like is displayed.

The light-source control unit 204 controls the light-source driving unit 406, to control an operating state of the first light source 103A and the second light source 103B. The light-source control unit 204 controls the first light source 103A and the second light source 103B such that the first light source 103A and the second light source 103B emit the detection light in different timings.

The image-data acquiring unit 206 acquires image data of the eyeball 111 of the subject that is acquired by the stereo camera device 102 including the first camera 102A and the second camera 102B, from the stereo camera device 102 through the input/output unit 302.

The input-data acquiring unit 208 acquires input data that is generated as the input device 60 is operated, from the input device 60 through the input/output unit 302.

The position detecting unit 210 detects position data of a pupil center based on the image data of the eyeball 111 acquired by the image-data acquiring unit 206. Moreover, the position detecting unit 210 detects position data of a corneal reflection center based on the image data of the eyeball 111 acquired by the image-data acquiring unit 206. The pupil center is a center of the pupil 112. The corneal reflection center is a center of the corneal reflection image 113. The position detecting unit 210 detects the position data of the pupil center and the position data of the corneal reflection center for the respective left and right eyeballs 111 of the subject.

The curvature-center calculating unit 212 calculates position data of a corneal curvature center of the eyeball 111 based on the image data of the eyeball 111 acquired by the image-data acquiring unit 206.

The gaze-point detecting unit 214 detects position data of a gaze point of the subject based on the image data of the eyeball 111 acquired by the image-data acquiring unit 206. In the present embodiment, the position data of a gaze point is position data of an intersection of a line-of-sight vector of the subject that is defined by the three-dimensional global coordinates and the display screen 101S of the display device 101. The gaze-point detecting unit 214 detects a line-of-sight vector of each of the left and right eyeballs 111 of the subject based on the position data of the pupil center and the position data of the corneal curvature center acquired from the image data of the eyeball 111. After the line-of-sight vector is detected, the gaze-point detecting unit 214 detects position data of a gaze point that indicates an intersection of the line-of-sight vector and the display screen 101S.

The region setting unit 216 sets a corresponding region that corresponds to each of the plural objects on the display screen 101S of the display device 101. The region setting unit 216 sets, as a specified region, a corresponding region that corresponds to an object to be looked at by the subject out of the plural objects.

The determining unit 218 determines whether a gaze point is present in each of plural corresponding regions in the non-display period in which the non-display operation is performed, and outputs determination data. The determining unit 218 determines whether a gaze point is present in each of the corresponding regions, for example, every fixed time. As for the fixed time, for example, it can be a period (for example, every 50 [msec]) of a frame synchronization signal output from the first camera 102A and the second camera 102B.

The arithmetic unit 220 calculates region data that indicates a corresponding region in which a gaze point is detected in the non-display period out of the plural corresponding regions based on the determination data of the determining unit 218. Moreover, the arithmetic unit 220 calculates presence time data that indicates presence time in which a gaze point is present in the plural corresponding regions in the non-display period based on the determination data of the determining unit 218. Furthermore, the arithmetic unit 220 calculates reaching time data that indicates reaching time of a gaze point until the gaze point reaches the specified region from the start time of the non-display period based on the determination data of the determining unit 218.

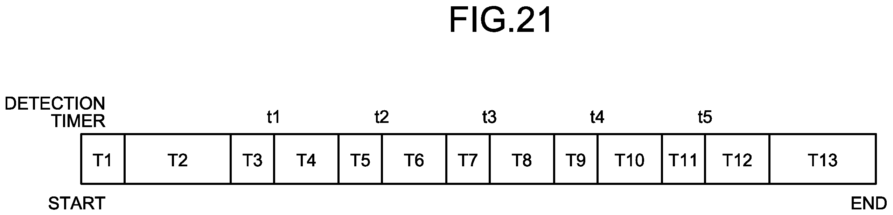

The arithmetic unit 220 has a management timer that manages reproduction time of an image, and a detection timer that detects elapsed time from when an image is displayed on the display screen 101S. The arithmetic unit 220 can detects an image displayed on the display screen 101S is an image of which period out of plural periods (refer to period T1 to T13 in FIG. 21) in a time chart. Moreover, the arithmetic unit 220 counts the number of times of determination of determining that a gaze point is present in each of corresponding regions. The arithmetic unit 220 has a counter that counts the number of time of determination for each corresponding region. Furthermore, the arithmetic unit 220 has a counter that counts reaching time data that indicates reaching time from a start time of the non-display operation until when a gaze point first reaches the specified region.

The evaluating unit 224 calculates evaluation data of a subject based on at least region data. The evaluation data is data that indicates how much the subject remembers positions of plural objects displayed on the display screen 101S in the display operation. The evaluating unit 224 can calculate evaluation data based on the region data and presence time data. Moreover, the evaluating unit 224 can calculate evaluation data based on the region data, the presence time data, and the reaching time data. In this case, for example, the evaluation data may be calculated, for example, assigning a heavier weight to the presence time data than the reaching time data.

The storage unit 222 stores therein the region data, the presence time data, the reaching time data, and the evaluation data described above. Furthermore, the storage unit 222 stores therein an evaluation program that causes a computer to perform: a process of acquiring image data of an eyeball of a subject; a process of detecting position data of a gaze point of the subject based on the image data; a process of performing the display operation in which plural objects are displayed on a display screen and the non-display operation in which the objects are hidden in predetermined timing after the display operation is started; a process of setting plural corresponding regions that correspond to respective objects on the display screen; a process of determining, based on the position data of a gaze point, whether a gaze point is present in respective corresponding regions in the non-display period in which the non-display operation is performed and of outputting determination data; a process of respectively calculating, based on the determination data, region data that indicates a corresponding region in which a gaze point is detected in the non-display period among the corresponding regions; a process of calculating evaluation data of the subject based on the region data; and a process of outputting the evaluation data.

The output control unit 226 outputs data to at least one of the display device 101, the output device 50, and the voice output device 70. In the present embodiment, the output control unit 226 displays the region data and the time data calculated by the arithmetic unit 220 on the display device 101 or the output device 50. Moreover, the output control unit 226 displays the position data of a gaze point of each of the left and right eyeballs 111 of the subject on the display device 101 or the output device 50. Furthermore, the output control unit 226 displays the evaluation data output from the evaluating unit 224 on the display device 101 or the output device 50.

Next, an overview of processing of the curvature-center calculating unit 212 according to the present embodiment is described. The curvature-center calculating unit 212 calculates position data of a corneal curvature center of the eyeball 111 based on image data of the eyeball 111.

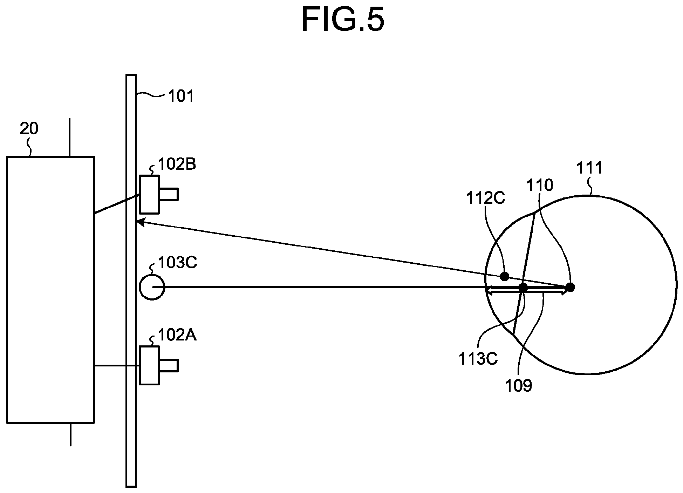

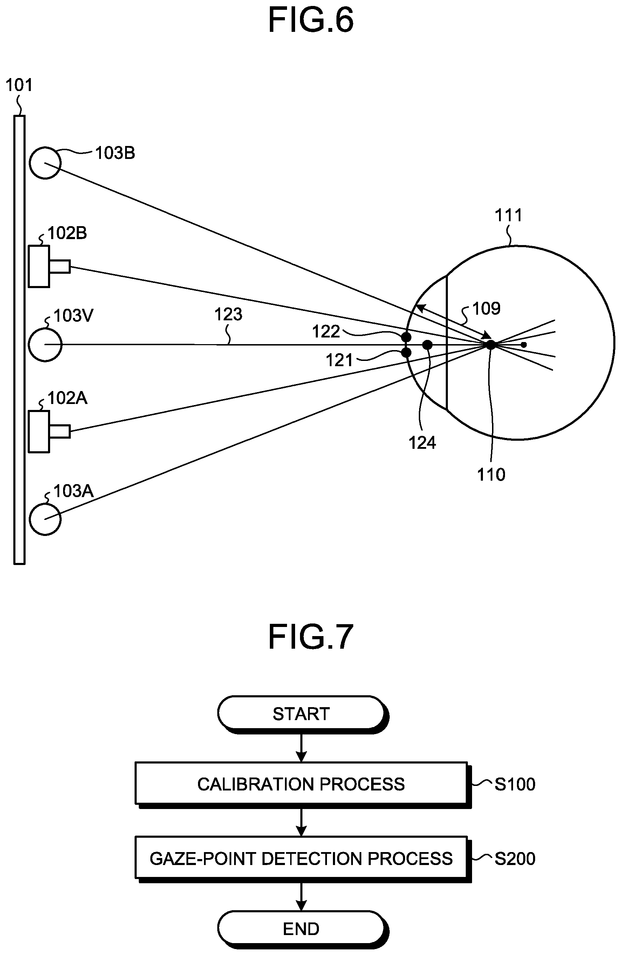

FIG. 5 and FIG. 6 are schematic diagrams for explaining a method of calculating position data of a corneal curvature center 110 according to the present embodiment. FIG. 5 illustrates an example in which the eyeball 111 is illuminated by one light source 103C. FIG. 6 illustrates an example in which the eyeball 111 is illuminated by the first light source 103A and the second light source 103B.

First, the example in FIG. 5 is described. The light source 103C is arranged between the first camera 102A and the second camera 102B. A pupil center 112C is a center of the pupil 112. A corneal reflection center 113C is a center of the corneal reflection image 113. In FIG. 5, the pupil center 112C indicates a pupil center when the eyeball 111 is illuminated by a single unit of the light source 103C. The corneal reflection center 113C indicates a corneal reflection center when the eyeball 111 is illuminated by one unit of the light source 103C.

The corneal reflection center 113C is present on a straight line connecting the light source 103C and the corneal curvature center 110. The corneal reflection center 113C is positioned at a middle point between a corneal surface and the corneal curvature center 110. A corneal curvature radius 109 is a distance between the corneal surface and the corneal curvature center 110.

The position data of the corneal reflection center 113C is detected by the stereo camera device 102. The corneal curvature center 110 is present on a straight line connecting the light source 103C and the corneal reflection center 113C. The curvature-center calculating unit 212 calculates position data that indicates a position at which a distance from the corneal reflection center 113C on the straight line becomes a predetermined value, as position data of the corneal curvature center 110. The predetermined value is a value determined in advance from a general curvature radius value of a cornea, or the like, and is stored in the storage unit 222.

Next, the example in FIG. 6 is described. In the present embodiment, the first camera 102A and the second light source 103B; and the second camera 102B and the first light source 103A are arranged at bilaterally symmetrical positions relative to a straight line passing through a meddle position between the first camera 102A and the second camera 102B. It can be regarded that a virtual light source 103V is present at the middle position between the first camera 102A and the second camera 102B.

A corneal reflection center 121 indicates a corneal reflection center in an image obtained by imaging the eyeball 111 by the second camera 102B. A corneal reflection center 122 indicates a corneal reflection center in an image obtained by imaging the eyeball 111 by the first camera 102A. A corneal reflection center 124 indicates a corneal reflection center corresponding to the virtual light source 103V.

Position data of the corneal reflection center 124 is calculated based on position data of the corneal reflection center 121 and position data of the corneal reflection center 122 acquired by the stereo camera device 102. The stereo camera device 102 detects position data of the corneal reflection center 121 and position data of the corneal reflection center 122 in a three-dimensional local coordinate system defined for the stereo camera device 102. For the stereo camera device 102, camera calibration by stereo calibration method is performed in advance, and a conversion parameter to convert a three-dimensional local coordinate system of the stereo camera device into a three-dimensional global coordinate system is calculated. The conversion parameter is stored in the storage unit 222.

The curvature-center calculating unit 212 converts the position data of the corneal reflection center 121 and the position data of the corneal reflection center 122 acquired by the stereo camera device 102 into position data in the three-dimensional global coordinate system by using the conversion parameter. The curvature-center calculating unit 212 calculates position data of the corneal reflection center 124 in the three-dimensional global coordinate system based on the position data of the corneal reflection center 121 and the position data of the corneal reflection center 122 defined by the three-dimensional global coordinate system.

The corneal curvature center 110 is present on a straight line 123 connecting the virtual light source 103V and the corneal reflection center 124. The curvature-center calculating unit 212 calculates position data that indicates a position at which a distance from the corneal reflection center 124 on the straight line 123 becomes a predetermined value, as position data of the corneal curvature center 110. The predetermined value is a value determined in advance from a general curvature radius value of a cornea, and is stored in the storage unit 222.

As described, also when two light sources are used, the corneal curvature center 110 is calculated by a method similar to the method in the case in which a single light source is used.

The corneal curvature radius 109 is a distance between a corneal surface and the corneal curvature center 110. Therefore, by calculating the position of the corneal surface and the corneal curvature center 110, the corneal curvature radius 109 is calculated.

### Eye Tracking Method

Next, an example of an eye tracking method according to the present embodiment is described. FIG. 7 is a flowchart showing an example of the eye tracking method according to the present embodiment. In the present embodiment, the calibration process including the calculation process of position data of the corneal curvature center 110, and the calculation process of distance data between the pupil center 112C and the corneal curvature center 110 (step S100), and the gaze-point detection process (step S200) are performed.

### Calibration Process

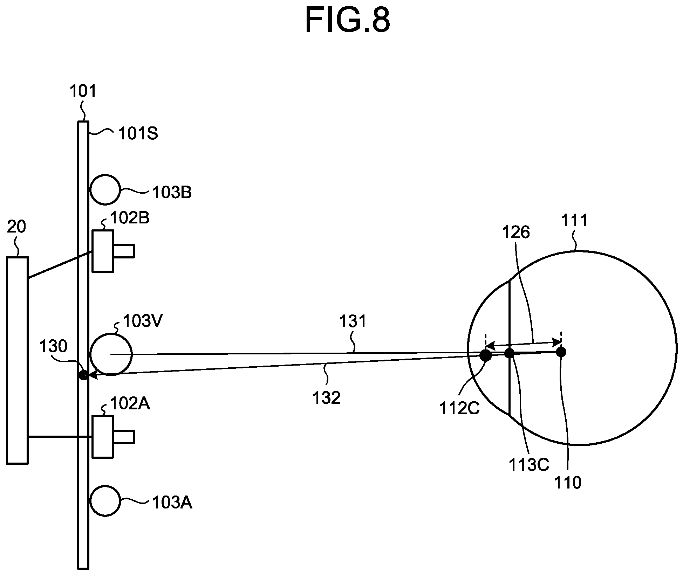

The calibration process (step S100) is described. FIG. 8 is a schematic diagram for explaining an example of the calibration process according to the present embodiment. The calibration process includes calculation of position data of the corneal curvature center 110, and calculation of a distance 126 between the pupil center 112C and the corneal curvature center 110.

A target position 130 to be looked at by the subject is set. The target position 130 is defined in the three-dimensional global coordinates. In the present embodiment, the target position 130 is set, for example, at a center position of the display screen 101S of the display device 101. The target position 130 may be set at an end position of the display screen 101S.

The display control unit 202 displays a target image at the set target position 130. Thus, the subject is more likely to look at the target position.

A straight line 131 is a straight line connecting the virtual light source 103V and the corneal reflection center 113C. A straight line 132 is a straight line connecting the target position 130 and the pupil center 112C. The corneal curvature center 110 is an intersection of the straight line 131 and the straight line 132. The curvature-center calculating unit 212 can calculate position data of the corneal curvature center 110 based on the position data of the virtual light source 103V, the position data of the target position 130, the position data of the pupil center 112C, and the position data of the corneal reflection center 113C.

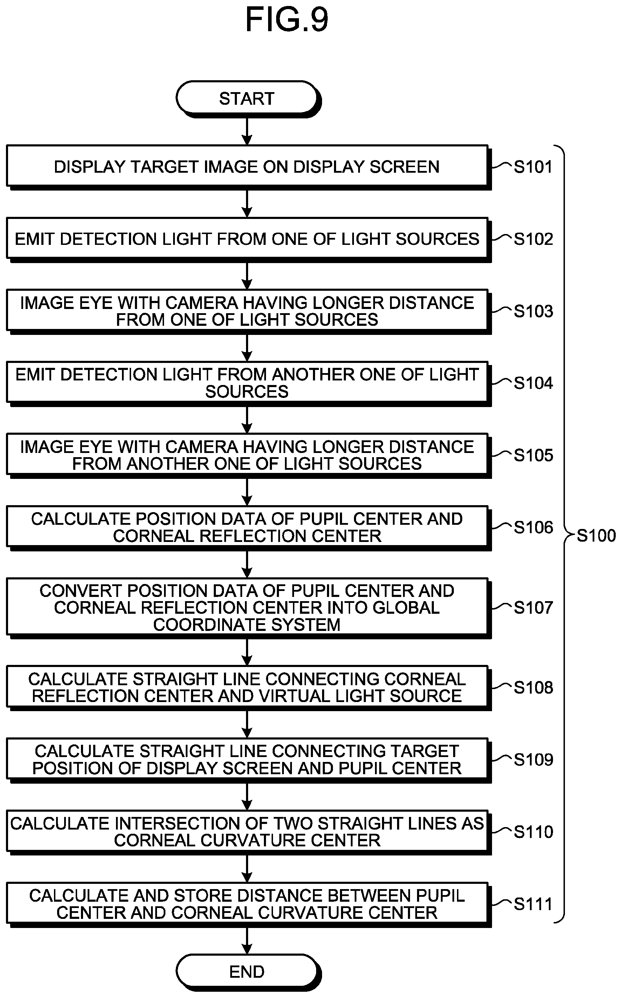

FIG. 9 is a flowchart showing an example of the calibration process (step S100) according to the present embodiment. The output control unit 226 displays a target image on the display screen 101S of the display device 101 (step S101). The subject can look at the target position 130 by looking at the target image.

Next, the light source control unit 204 controls the light-source driving unit 406 to emit the detection light from one of the light sources out of the first light source 103A and the second light source 103B (step S102). The stereo camera device 102 images an eyeball of the subject with a camera having a longer distance from the light source from which the detection light is emitted out of the first camera 102A and the second camera 102B (step S103).

Next, the light-source control unit 204 controls the light-source driving unit 406 to emit the detection light from the other one of the light sources out of the first light source 103A and the second light source 103B (step S104). The stereo camera device 102 images an eyeball of the subject with a camera having a longer distance from the light source from which the detection light is emitted out of the first camera 102A and the second camera 102B (step S105).

The pupil 112 is detected by the stereo camera device 102 as a dark portion, and the corneal reflection image 113 is detected by the stereo camera device 102 as a bright portion. That is, an image of the pupil 112 acquired by the stereo camera device 102 is to be a low intensity image, and an image of the corneal reflection image 113 is to be a high intensity image. The position detecting unit 210 can detect position data of the pupil 112 and position data of the corneal reflection image 113 based on the intensity of the acquired image. Moreover, the position detecting unit 210 calculates position data of the pupil center 112C based on image data of the pupil 112. Furthermore, the position detecting unit 210 calculates position data of the corneal reflection center 113C based on image data of the corneal reflection image 113 (step S106).

The position data detected by the stereo camera device 102 is position data defined by the three-dimensional local coordinate system. The position detecting unit 210 subjects the position data of the pupil center 112C and the position of the corneal reflection center 113C detected by the stereo camera device 102 to coordinate conversion by using the conversion parameter stored in the storage unit 222, to calculate position data of the pupil center 112C and position data of the corneal reflection center 113C defined by the three-dimensional global coordinate system (step S107).

The curvature-center calculating unit 212 calculates the straight line 131 connecting the corneal reflection center 113C defined by the global coordinate system and the virtual light source 103V (step S108).

Next, the curvature-center calculating unit 212 calculates the straight line 132 connecting the target position 130 set on the display screen 101S of the display device 101 and the pupil center 112C (step S109). The curvature-center calculating unit 212 calculates an intersection of the straight line 131 calculated at step S108 and the straight line 132 calculated at step S109, and determines this intersection as the corneal curvature center 110 (step S110).

The curvature-center calculating unit 212 calculates a distance 126 between the pupil center 112C and the corneal curvature center 110, and stores it in the storage unit 222 (step S111). The stored distance is used to calculate the corneal curvature center 110 in the gaze point detection at step S200.

### Gaze-Point Detection Process

Next, the gaze-point detection process (step S200) is described. The gaze-point detection process is performed after the calibration process. The gaze-point detecting unit 214 calculates a line-of-sight vector and position data of a gaze point of the subject based on image data of the eyeball 111.

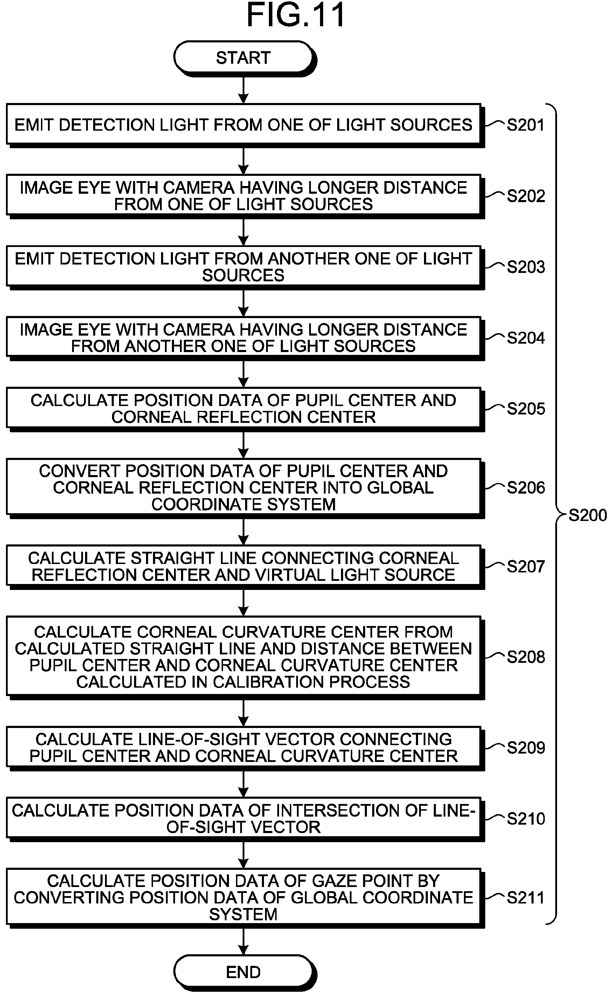

FIG. 11 is a schematic diagram for explaining an example of the gaze-point detection process according to the present embodiment. The gaze-point detection process includes correction of a position of the corneal curvature center 110 by using the distance 126 between the pupil center 112C and the corneal curvature center 110 acquired in the calibration process (step S100), and calculation of a gaze point by using corrected position data of the corneal curvature center 110.

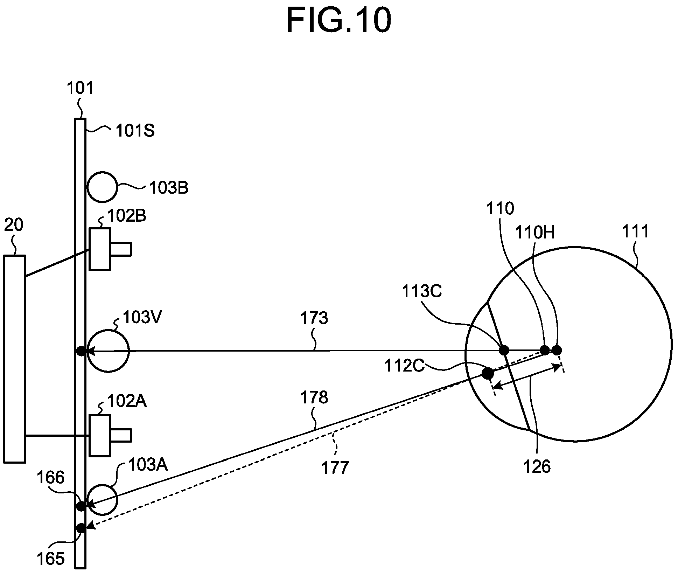

In FIG. 10, a gaze point 165 indicates a gaze point that is acquired from the corneal curvature center calculated by using a general curvature radius value. A gaze point 166 indicates a gaze point that is acquired from a corneal curvature center calculated by using the distance 126 acquired in the calibration process.

The pupil center 112C indicates a pupil center that is calculated in the calibration process, and the corneal reflection center 113C indicates a corneal reflection center that is calculated in the calibration process.

A straight line 173 is a straight line connecting the virtual light source 103V and the corneal reflection center 113C. The corneal curvature center 110 is a position of a corneal curvature center calculated from a general curvature radius value.

The distance 126 is a distance between the pupil center 112C and the corneal curvature center 110 calculated by the calibration process.

A corneal curvature center 110H indicates a position of a corrected corneal curvature center obtained by correcting the corneal curvature center 110 by using the distance 126.

The corneal curvature center 110H is calculated based on the facts that the corneal curvature center 110 is present on the straight line 173, and that the distance between the pupil center 112C and the corneal curvature center 110 is the distance 126. Thus, a line-of-sight 177 that is calculated when a general curvature radius value is used is corrected to a line-of-sight 178. Moreover, the gaze point on the display screen 101S of the display device 101 is corrected from the gaze point 165 to the gaze point 166.

FIG. 11 is a flowchart showing an example of the gaze-point detection process (step S200) according to the present embodiment. Because processes from step S201 to step S207 shown in FIG. 11 are similar to the processes from step S102 to step S108 shown in FIG. 9, explanation thereof is omitted.

The curvature-center calculating unit 212 calculates a position that is on the straight line 173 calculated at step S207, and at which a distance from the pupil center 112C is equal to the distance 126 calculated in the calibration process, as the corneal curvature center 110H (step S208).

The gaze-point detecting unit 214 calculates a line-of-sight vector connecting the pupil center 112C and the corneal curvature center 110H (step S209). The line-of-sight vector indicates a direction of sight toward which the subject is looking. The gaze-point detecting unit 214 calculates position data of an intersection of the line-of-sight vector and the display screen 101S of the display device 101 (step S210). The position data of the intersection of the line-of-sight vector and the display screen 101S of the display device 101 is position data of a gaze point of the subject on the display screen 101S defined by the three-dimensional global coordinate system.

The gaze-point detecting unit 214 converts position data of the gaze point defined by the three-dimensional global coordinate system to position data on the display screen 101S of the display device 101 defined by a two-dimensional coordinate system (step S211). Thus, the position data of the gaze point on the display screen 101S of the display device 101 at which the subject looks is calculated.

Next, an evaluation method according to the present embodiment is described. In the present embodiment, the eye tracking device 100 is used as an evaluation device that evaluates, for example, a subject of interest of the subject. In the following description, the eye tracking device 100 can be referred to as evaluation device 100 as appropriate.

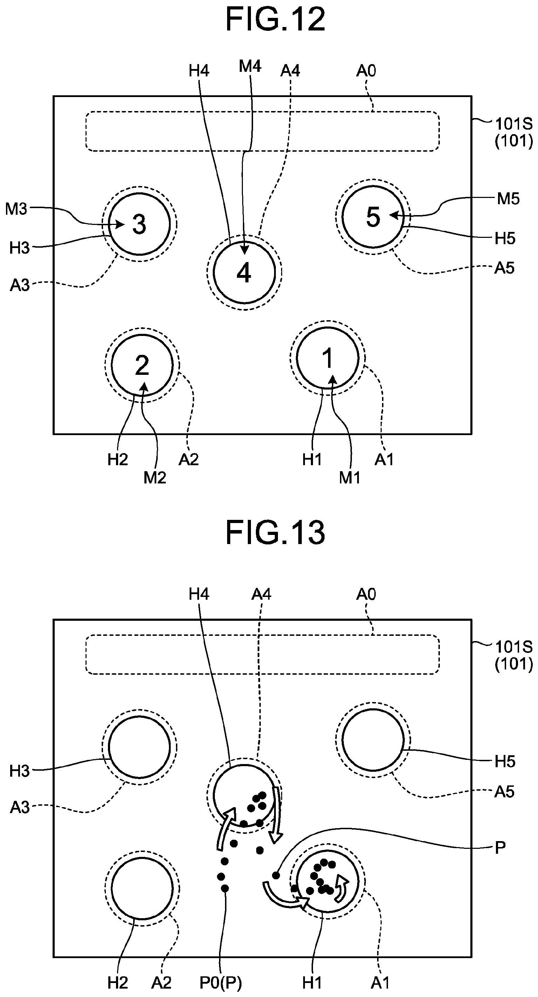

FIG. 12 is a diagram illustrating an example of an image displayed on the display device 101 by the display control unit 202. As illustrated in FIG. 12, the display control unit 202 displays, for example, five objects M1 to M5 on the display screen 101S of the display device 101. The display control unit 202 displays the objects M1 to M5 on the display screen 101S, for example, in a separated manner from one another.

The objects M1 to M5 are, for example, images each indicating a number. The object M1 indicates "1", the object M2 indicates "2", the object M3 indicates "3", the object M4 indicates "4", and the object M5 indicates "5". In FIG. 12, images each indicating a number are shown as the objects M1 to M5 as an example, but it is not limited thereto. As these objects, images of other kinds, for example, images indicating alphabets, such as "A", "B", and "C", images indicating hiragana characters, such as "a", "i", and "u", images indicating katakana characters, such as "a", "i", and "u", images indicating fruits, such as "apple", "orange", and "banana", or the like may be used as long as the images are distinguishable from one another.

Moreover, the region setting unit 216 sets corresponding regions A1 to A5 on the display screen 101S. The region setting unit 216 sets the corresponding regions A1 to A5 to the respectively corresponding objects M1 to M5. In the example illustrated in FIG. 12, the region setting unit 216 sets the corresponding regions A1 to A5 to be, for example, circular shapes in equal sizes to be portions surrounding the objects M1 to M5.

The corresponding regions A1 to A5 are not necessarily required to be the same shape in the same size, but shapes and sizes may be different from one another. Moreover, the corresponding regions A1 to A5 are not limited to be in a circular shape, but may be in a polygonal shape, such as a triangular shape, a rectangular shape, and a star shape, or may be in other shapes, such as an oval shape, and the like. For example, the corresponding regions A1 to A5 may be in a shape along an outline of each of the objects M1 to M5. Furthermore, the region setting unit 216 may set the corresponding regions A1 to A5 to a portion including only a part of the respective objects M1 to M5.

In the present embodiment, the display control unit 202 displays range regions H1 to H5 on the display screen 101S of the display device 101. The range regions H1 to H5 are regions indicating ranges of the respective corresponding regions A1 to A5. Displaying the range regions H1 to H5 on the display screen 101S makes it easy for the subject to grasp the ranges of the corresponding regions A1 to A5. The range regions H1 to H5 can be formed, for example, in an identical shape to the corresponding regions A1 to A5, that is in a similar shape to the corresponding regions A1 to A5, but not limited thereto. The range regions H1 to H5 are set, for example, in a range included in the corresponding regions A1 to A5 but, not limited thereto, may be set outside the regions of the corresponding regions A1 to A5. Furthermore, the range regions H1 to H5 may not be displayed.

Moreover, the display control unit 202 displays an instruction to the subject in an instruction region A0 on an upper side of the display screen 101S. The instruction region A0 is to display contents of various instructions when instructing the subject to remember types and positions of the objects M1 to M5, when instructing the subject to look at a specific region that is a predetermined corresponding region out of the corresponding regions A1 to A5, that is, instructing to look at the specific region, or the like.

FIG. 13 is a diagram illustrating an example of movement of a gaze point of the subject, and is a diagram illustrating an example of a gaze point that is displayed on the display device 101 by the output control unit 226. In FIG. 13, gaze points in the case in which the corresponding regions A1, A4 are looked at are shown. The output control unit 226 displays plot points P that indicate position data of the gaze point of the subject on the display device 101. Detection of the position data of a gaze point is performed, for example, in a cycle of a frame synchronization signal output from the first camera 102A and the second camera 102B (for example, every 50 [msec]). The first camera 102A and the second camera 102B shoot images in synchronization with each other. Therefore, it is indicated that a region in which the plot points P are densely present in the display screen 101S is looked at more than others by the subject. Moreover, it is indicated that a region in which more plot points P are present is looked at by the subject for longer time.

FIG. 13 illustrates a case in which the objects M1 to M5 are not displayed but the range regions H1 to H5 are displayed. In this case, the gaze point P first moves from an initial position P0 toward the corresponding region A4 and the range region H4 (an upward direction in FIG. 13), and enters the corresponding region A4 and the range region H4. Thereafter, after moving inside the corresponding region A4 and the range region H4, the gaze point moves out of the corresponding region A4 and the range region H4 and moves toward the corresponding region A1 and the range region H1 (a lower right side in FIG. 13), to enter the corresponding region A1 and the range region H1. In the example in FIG. 13, it is indicated that as a result of an action of the subject of shifting a line of sight from the range region H4 to the range region H1 out of the range regions H1 to H5 displayed on the display screen 101S, the gaze point P enters the corresponding region A4 and the corresponding region A1 with movement of the line of sight of the subject.

It has been known that symptoms of cranial nerve disease and disorder affect memory. If the subject is not a person with cranial nerve disease and disorder, the subject can memorize types and positions of the objects M1 to M5 in short time. On the other hand, when the subject is a person with cranial nerve disease and disorder, there is a case of being disable to memorize types and positions of the objects M1 to M5 in a short time, and a case of being able to memorize them but forgetting them soon.

Therefore, for example, by performing following procedures, evaluation of the subject is possible. First, the subject is caused to memorize types and positions of the objects M1 to M5 in a state in which the objects M1 to M5 are displayed on the display screen 101S. Thereafter, it is brought to a state in which the objects M1 to M5 are not displayed on the display screen 101S, and the subject is instructed to look at one position out of the objects M1 to M5. In this case, it is possible to evaluate the subject by detecting which corresponding region the subject first looks at out of the corresponding regions A1 to A5 corresponding to the objects M1 to M5 by the subject, or by detecting whether the subject can look at it stably for long time.

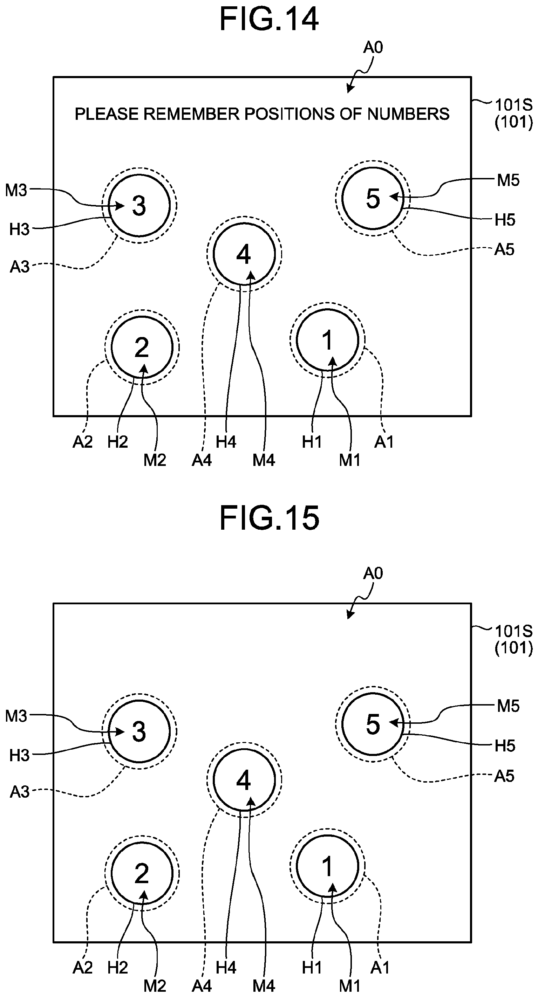

FIG. 14 to FIG. 20 are diagrams illustrating an example of an image displayed on the display screen 101S by the display control unit 202 according to the present embodiment. FIG. 21 is a time chart showing a time at which each image is displayed. When an image is reproduced by the display control unit 202, first, as illustrated in FIG. 14, the objects M1 to M5 and the range regions H1 to H5, an instruction telling, "please remember positions of numbers" in the instruction region A0 are displayed on the display screen 101S for a predetermined period (period T1 in FIG. 21).

After the period T1 elapses, the instruction in the instruction region A0 is deleted from the display screen 101S as illustrated in FIG. 15. Therefore, the range regions H1 to H5 of the objects M1 to M5 are displayed (display operation) on the display screen 101S for predetermined time (period T2 in FIG. 21). The period T2 is a display period in which the display operation is performed. Because the objects M1 to M5 are displayed also in the period T1 described above, the period T1 may be included in the display period. In the period T2, remaining time may be displayed in the instruction region A0.

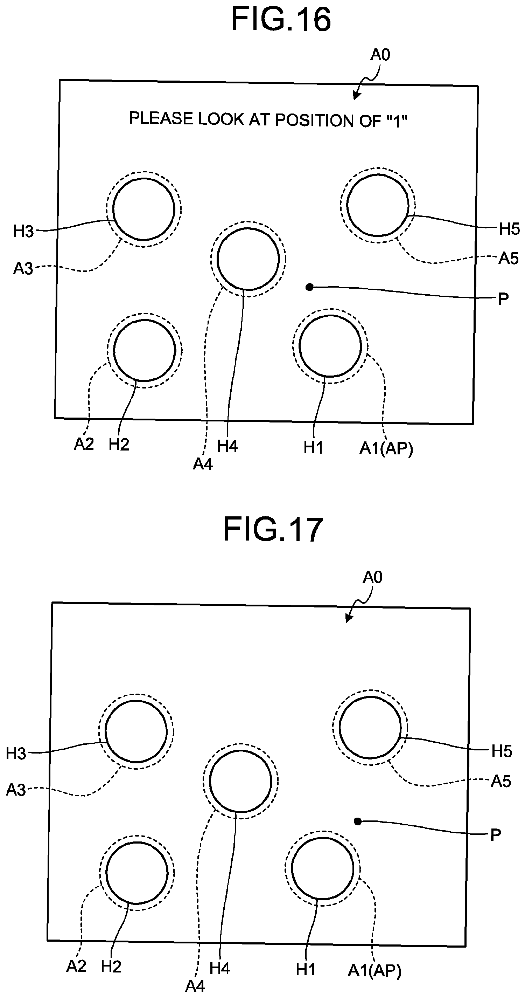

After the period T2 elapses, the display of the objects M1 to M5 is deleted from the display screen 101S as illustrated in FIG. 16. Therefore, the range regions H1 to H5 and an instruction telling "please look at a position of '1'" are displayed on the display screen 101S for a predetermined period (period T3 in FIG. 21) in a state in which the objects M1 to M5 are not displayed.

After the period T3 elapses, the instruction in the instruction region A1 is deleted from the display screen 101S as illustrated in FIG. 17. Therefore, the range regions H1 to H5 are displayed for a predetermined period (period T4 in FIG. 21) on the display screen 101S in a state in which the objects M1 to M5 are not displayed (non-display operation). The period T4 is a non-display period in which the non-display operation is performed. A start time of the period T4 is a start time t1 of the non-display period (refer to FIG. 21). Because the objects M1 to M5 are not displayed also in the period T3 described above, the period T3 may be included in the non-display period. In this case, a start time of the period T3 is the start time t1 of the non-display period. In the period T3 and the period T4, the output control unit 226 may display the plot point P indicating position data of a gaze point of the subject on the display screen 101S. The region setting unit 216 sets the corresponding region A1 corresponding to the object M1 (number "1") as a specified region AP, although not displayed.

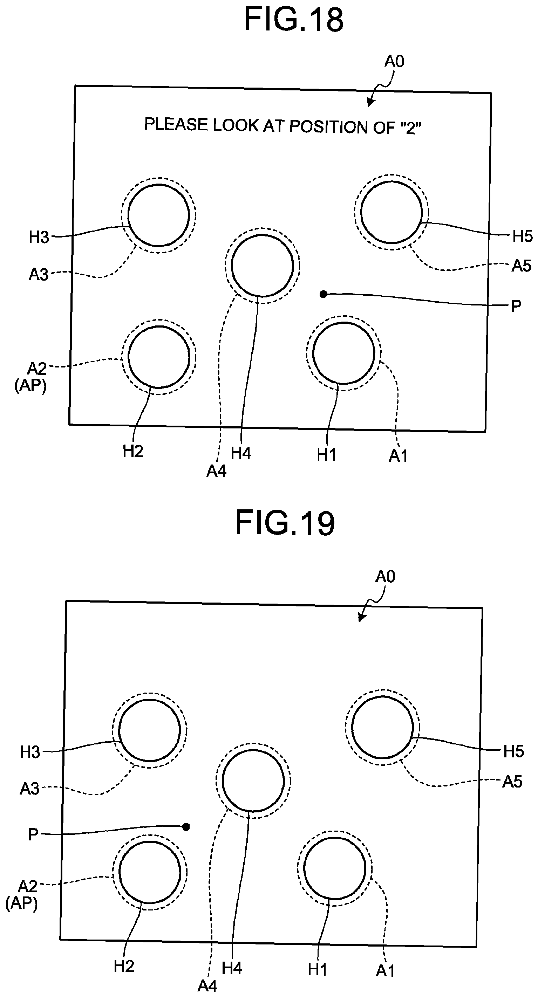

After the period T4 elapses, an instruction telling "please look at a position of '2'" is displayed on the display screen 101S as illustrated in FIG. 18. Therefore, the range regions H1 to H5 and the instruction telling "please look at a position of '2'" are displayed for predetermined period (period T5 in FIG. 21) on the display screen 101S in a state in which the objects M1 to M5 are not displayed. After the period T5 elapses, the instruction in the instruction region A0 is deleted from the display screen 101S as illustrated in FIG. 19. Therefore, the range regions H1 to H5 are displayed for a predetermined period (period T6 in FIG. 21) on the display screen 101S in a state in which the objects M1 to M5 are not displayed. The period T6 is a non-display period in which the non-display operation is performed. A start time of the period T6 is a start time t2 of the non-display period (refer to FIG. 21). Because the objects M1 to M5 are not displayed also in the period T5 described above, the period T5 may be included in the non-display period. In this case, a start time of the period T5 is the stat time t2 of the non-display period. In the period T6, the output control unit 226 may display the plot point P indicating position data of a gaze point of the subject on the display screen 101S. The region setting unit 216 sets the corresponding region A2 corresponding to the object M2 (number "2") as the specified region AP in the period T5 and the period T6, although not displayed.

Hereinafter, illustration of the display screen 101S is omitted, but after the period T6 elapses, the range regions H1 to H5 and an instruction telling "please look at a position of '3'" are displayed on the display screen 101S for predetermined time (period T7 in FIG. 21) in a state in which the objects M1 to M5 are not displayed. After the period T7 elapses, the instruction in the instruction region A0 is deleted from the display screen 101S, and the range region H1 to H5 are displayed on the display screen 101S for a predetermined period (period T8 in FIG. 21) in a state in which the objects M1 to M5 are not displayed (non-display operation). The region setting unit 216 sets the corresponding region A3 that corresponds to the object M3 (number "3") as the specified region AP in the period T7 and the period T8, although not displayed on the display screen 101S.

After the period T8 elapses, the range regions H1 to H5 and an instruction telling "please look at a position of '4'" are displayed on the display screen 101S for predetermined time (period T9 in FIG. 21) in a state in which the objects M1 to M5 are not displayed. After the period T9 elapses, the instruction in the instruction region A0 is deleted from the display screen 101S, and the range region H1 to H5 are displayed on the display screen 101S for a predetermined period (period T10 in FIG. 21) in a state in which the objects M1 to M5 are not displayed (non-display operation). The region setting unit 216 sets the corresponding region A4 that corresponds to the object M4 (number "4") as the specified region AP in the period T9 and the period T10, although not displayed on the display screen 101S.

After the period T10 elapses, the range regions H1 to H5 and an instruction telling "please look at a position of '5'" are displayed on the display screen 101S for predetermined time (period T11 in FIG. 21) in a state in which the objects M1 to M5 are not displayed. After the period T11 elapses, the instruction in the instruction region A0 is deleted from the display screen 101S, and the range region H1 to H5 are displayed on the display screen 101S for a predetermined period (period T12 in FIG. 21) in a state in which the objects M1 to M5 are not displayed (non-display operation). The region setting unit 216 sets the corresponding region A5 that corresponds to the object M5 (number "5") as the specified region AP in the period T11 and the period T12, although not displayed on the display screen 101S.

The respective periods T8, T10, T12 described above are non-display periods in which the non-display operation is performed. Start times of the periods T8, T10, T12 are start times t3, t4 and t5 of the non-display periods (refer to FIG. 21). Because the objects M1 to M5 are not displayed also in the periods T7, T9, T11 described above, the periods T7, T9, T11 may be included in the non-display period. In this case, start times of the period T7, T9, T11 are the start times t3, t4, and t5 in the non-display period. In the periods T8, T10, and T12, the output control unit 226 may display the plot point P indicating position data of a gaze point of the subject on the display screen 101S.

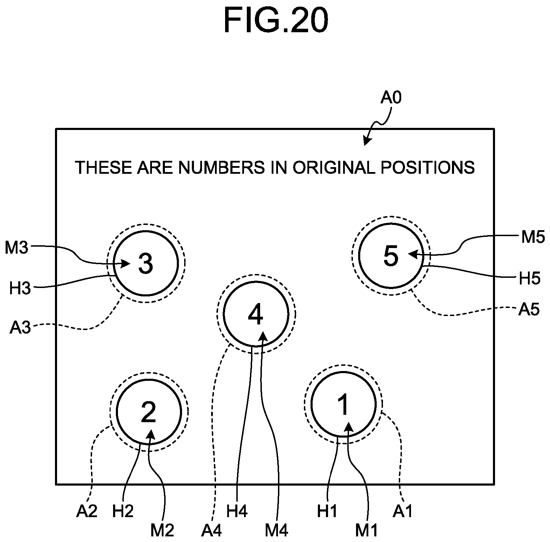

After the period T12 elapses, as illustrated in FIG. 20, the objects M1 to M5 are displayed on the display screen 101S, and an instruction indicating, "these are numbers in original positions", or the like is displayed in the instruction region A0 (period T13 in FIG. 21). After the period T13 elapses, reproduction of the image is finished. A message indicating an end of the image may be displayed on the display screen 101S in the period T13.

If the subject is not a person with cranial nerve disease and disorder, the subject can bring the eyepoint to a correct position based on the memory when instructed to look at one position out of the objects M1 to M5. On the other hand, if the subject is a person with cranial nerve disease or disorder, there is a case in which the subject cannot bring the eyepoint to a correct position when instructed to look at one position out of the objects M1 to M5.

In the above periods T4, T6, T8, T10, and T12, which are the non-display periods, the determining unit 218 determines whether a gaze point is present in the respective corresponding regions A1 to A5, and outputs determination data. Moreover, the arithmetic unit 220 calculates presence time data that indicates presence time in which the plot point P showing a gaze point is present in the respective corresponding regions A1 to A5, based on the determination data in the periods T4, T6, T8, T10, and T12, which are the non-display period. In the present embodiment, for example, the presence time includes first presence time in which a gaze point is present in the specified region AP out of the corresponding regions A1 to A5, and second presence time in which a gaze point is present in a corresponding region that is not the specified region AP. Therefore, the presence time data includes first presence-time data including the first presence time and second presence-time data including the second presence time. In the present embodiment, the first presence time (first presence-time data) and the second presence time (second presence-time data) can be a sum of values acquired in the respective periods T4, T6, T8, T10, and T12.

Furthermore, in the present embodiment, it is possible to estimate that a corresponding region determined more frequently that a gaze point is present therein has longer presence time in which a gaze point is present in the corresponding region. Therefore, in the present embodiment, the presence time data can be regarded as the number of times when the determining unit 218 determines that a gaze point is present in the non-display period for the corresponding regions A1 to A5. That is, the presence time data can be the number of the plot points P detected in each of the corresponding regions A1 to A5 in the non-display period. The arithmetic unit 220 can calculate the presence time data by using a count result of a counter that is provided in the determining unit 218.

In the present embodiment, the evaluating unit 224 can calculate evaluation data based on the region data, the presence time data, and the reaching time data in the following manner, for example.

First, a counter that is provided in the arithmetic unit 220 counts the first presence-time data, the second presence-time data, and the reaching time data in each of the periods T4, T6, T8, T10, and T12. Note that when counting the reaching time data, the counter performs counting based on a measurement flag. The measurement flag is set to either value of "0" or "1" by the arithmetic unit 220. When the value of the measurement flag is "0", the counter does not count the reaching time data. When the value of the measurement flag is "1", the counter counts the reaching time data. Herein, the counter value of the first presence-time data is CNTA, the counter value of the second presence-time data is CNTB, and the counter value of the reaching time data is CNTC. In the present embodiment, the counter value CNTA and the counter value CNTB are values obtained throughout the periods T4, T6, T8, T10, and T12. Moreover, the counter value CNTC is a value counted in each of the periods T4, T6, T8, T10, and T12.

In this case, the evaluation value to calculate evaluation data can be calculated as follows. For example, the evaluation value can be calculated by determining length of time in which a gaze point of the subject is present in the specified region AP. When the subject remembers a position of the object M1, time of looking at the specified region AP increases. The longer the presence time in which a gaze point is present in the specified region AP is, the larger the counter value CNTA becomes. Therefore, by determining whether a value of the counter value CNTA, which is the first presence-time data, is equal to or larger than a predetermined value, the evaluation value can be calculated. For example, when a value of the counter value CNTA is equal to or larger than the predetermined value, it can be evaluated that the subject is not likely to have cranial nerve disease or disorder. Moreover, when a value of the counter value CNTA is smaller than the predetermined threshold, it can be evaluated that the subject is likely to have cranial nerve disease or disorder.

As the predetermined value, for example, an average value of the counter value CNTA of subjects that are not a person with cranial nerve disease or disorder, a value that is set based on the average value, or the like can be used. Moreover, as the predetermined value, for example, a minimum value of the counter value CNTA of subjects that are not a person with neurological disorders may be used. In this case, the predetermined value may be set in advance by age and sex, and a value according to the age and the sex of a subject may be used.

Moreover, for example, an evaluation value can be calculated by Equation (1) below. $ANS 1 = CNTA / \left(CNTA+CNTB\right)$

In the above ANS1, a value of CNTA/(CNTA + CNTB) indicates a ratio of the counter value CNTA to a sum of the counter value CNTA and the counter value CNTB. That is, it indicates a rate of the first presence time in which a gaze point of the subject is present in the specified region AP. Hereinafter, ANS1 is referred to as a specified-region gaze rate.

A value of the specified-region gaze rate ANS1 takes a larger value as the counter value CNTA increases. That is, the value of the specified-region gaze rate ANS1 becomes a larger value as the first presence time increases in the period T4, which is the non-display period. Furthermore, the specified-region gaze rate ANS1 becomes 1, which is the maximum value, when the counter value CNTB is 0, that is, when the second presence time is 0.

In this case, the evaluation value can be calculated by determining whether the specified-region gaze rate ANS1 is equal to or larger than a predetermined value. For example, when the value of the specified-region gaze rate ANS1 is larger than the predetermined value, it can be evaluated that the subject is not likely to have cranial nerve disease or disorder. Moreover, when the specified-region gaze rate ANS1 is smaller than the predetermined value, it can be evaluated that the subject is likely to have cranial nerve disease or disorder.

As the predetermined value, for example, an average value of the specified-region gaze rate ANS1 of subjects that are not a person with cranial nerve disease or disorder, a value set based on the average value, or the like can be used. Furthermore, as the predetermined value, a minimum value of the specified-region gaze rate ANS1 of subjects that are not a person with cranial nerve disease or disorder may be used. In this case, the predetermined value may be set in advance by age and sex, and a value according to the age and the sex of a subject may be used.

Moreover, the evaluation value can be calculated by determining reaching time that indicated a time until a gaze point of the subject first reaches the specified region AP from the start time t1 in the non-display period, for example. If the subject remembers a position of the object M1, time until an eyepoint first reaches the specified region AP is to be short. The shorter the reaching time until an eyepoint reaches the specified region AP, the smaller the value of the counter value CNTC becomes. Therefore, the evaluation value can be calculated by determining whether a value of the counter value CNTC, which is the reaching time data, is equal to or smaller than a predetermined value. For example, when the counter value CNTC is equal to or larger than the predetermined value, it can be evaluated that the subject is not likely to have cranial nerve disease or disorder. Furthermore, when the counter value CNTC is smaller than the predetermined value, it can be evaluated that the subject is likely to have cranial nerve disease or disorder.

Moreover, for example, the evaluation value can be calculated by Equation (2) below.$ANS = ANS 1 \times K 1 + ANS 2 \times K 2$ (where ASN2 = K3 - CNTC)

In above Equation (2), a value ANS2 is a value obtained by subtracting the counter value CNTC, that is, the reaching time, from K3 to be a reference value. Hereinafter, ANS2 is referred to as a reaching-time evaluation value. As a constant K3, an average value of the counter value CNTC of subjects that are not a person with cranial nerve disease or disorder, a value set based on the average value, or the like can be used. Furthermore, as the constant K3, a minimum value of the counter value CNTC of subjects that are not a person with cranial nerve disease or disorder may be used. In this case, the constant K3 may be set in advance by age and sex, and a value according to the age and the sex of a subject may be used.

Constants K1, K2 are constants for weighting. When K1 > K2 in above Equation (2), an evaluation value ANS for which an influence of the specified-region gaze rate ANS1 is weighted, rather than an influence of the reaching-time evaluation value ANS2, can be calculated. When K1 < K2 in above Equation (2), an evaluation value ANS for which an influence of the reaching-time evaluation value ANS2 is weighted, rather than an influence of the specified-region gaze rate ANS1, can be calculated.

When a gaze point does not reach the specified region AP at a point of time when the non-display period ends, the counter value CNTC is to be a large value compared with other values. Therefore, it may be set such that the counter value CNTC takes a predetermined upper limit value when a gaze point does not reach the specified region Ap at a point of time when the non-display period ends.

It can be evaluated that the larger the evaluation value ANS indicated in above Equation (2) is, the longer the time in which a gaze point of the subject is present in the specified region AP is, and the shorter the time until the gaze point reaches the specified region AP is. Moreover, it can be evaluated that the smaller the evaluation value ANS is, the shorter the time in which a gaze point of the subject is present in the specified region AP is, and the longer the time until the gaze point reaches the specified region AP is. Therefore, the evaluation data can be calculated by determining whether the evaluation value ANS is equal to or larger than the predetermined value. For example, when the evaluation value ANS is equal to or larger than the predetermined value, it can be evaluated that the subject is not likely to have cranial nerve disease or disorder. Moreover, when the evaluation value ANS is smaller than the predetermined value, it can be evaluated that the subject is likely to have cranial nerve disease or disorder.

In the present embodiment, when the evaluating unit 224 outputs evaluation data, the output control unit 226 can cause the output device 50 to output, for example, text data indicating, "the subject is considered unlikely to have cranial nerve disease or disorder", text data indicating, "the subject is considered likely to have cranial nerve disease or disorder", and the like according to the evaluation data.

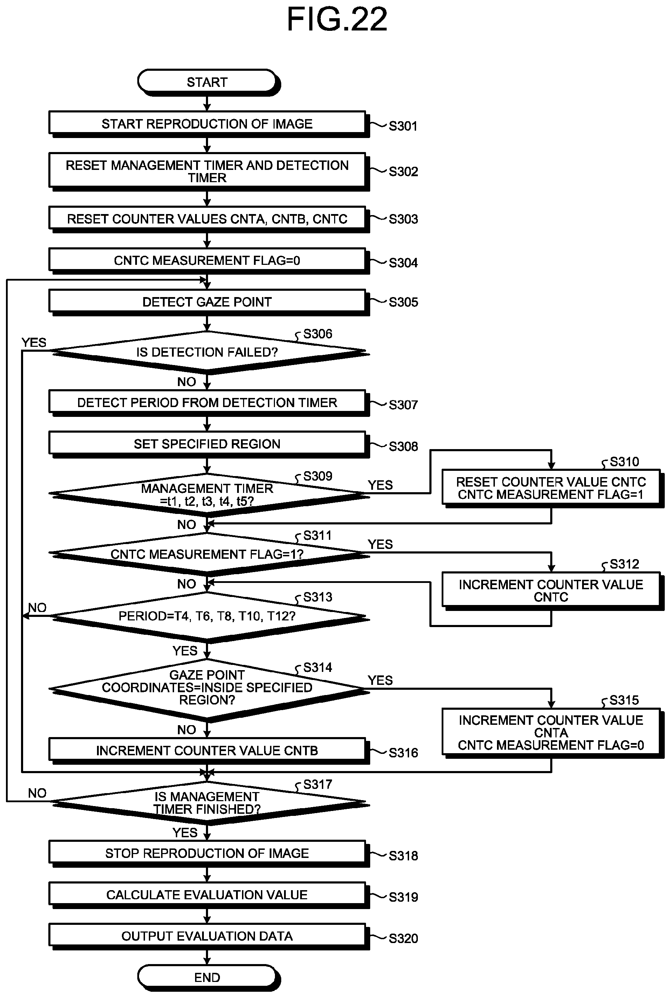

Next, an example of an evaluation method according to the present embodiment is described, referring to FIG. 22. FIG. 22 is a flowchart showing an example of the evaluation method according to the present embodiment. In the present embodiment, the display control unit 202 starts reproduction of an image (step S301). On the display screen 101S, images illustrated in FIG. 14 to FIG. 20 are displayed sequentially.

Moreover, the arithmetic unit 220 resets the management timer that manages reproduction time of an image, and a detection timer that detects a segment to which an image currently being reproduced belongs out of the periods T1 to the period T13 in the time chart in FIG. 21, and causes them to start measurement step S302). Furthermore, the determining unit 218 resets the counter values CNTA, CNTB, CNTC to 0, to start measurement (step S303). Moreover, the arithmetic unit 220 sets the value of the measurement flag of the counter value CNTC to 0 (step S304).

The gaze-point detecting unit 214 detects position data of a gaze point of the subject on the display screen 101S of the display device in every predetermined sampling cycle (for example, 50 [msec]) in a state in which the image displayed on the display device 101 is shown to the subject (step S305).

When the position data is detected (step S306: NO), the arithmetic unit 220 detects which period an image displayed on the display screen 101S corresponds to, out of the periods T1 to T13 based on the detection result of the detection timer (step S307). The region setting unit 216 sets the specified region AP from among the corresponding regions A1 to A5 based on the detection result of the arithmetic unit 220 (step S308). For example, when an image corresponding to the periods T3, T4 is displayed on the display screen 101S, the region setting unit 216 sets the corresponding region A1 to the specified region AP. When an image corresponding to the periods T5, T6 is displayed on the display screen 101S, the region setting unit 216 sets the corresponding region A2 to the specified region AP. When an image corresponding to the periods T7, T8 is displayed on the display screen 101S, the region setting unit 216 sets the corresponding region A3 to the specified region AP. When an image corresponding to the periods T9, T10 is displayed on the display screen 101S, the region setting unit 216 sets the corresponding region A4 to the specified region AP. When an image corresponding to the periods T11, T12 is displayed on the display screen 101S, the region setting unit 216 sets the corresponding region A5 to the specified region AP.

After the specified region AP is set, the arithmetic unit 220 determines whether it has come to the start times t1, t2, t3, t4, and t5 of the non-display operation based on a detection result of the management timer (step S309). When it is determined that it has come to the start times t1, t2, t3, t4, and t5 (step S309: YES), the arithmetic unit 220 resets the counter value CNTC of the reaching time data, and sets the value of the measurement flag of the reaching time data to "1" (step S310).

When it is determined that it has not come to the start times t1, t2, t3, t4, and t5 of the non-display operation (step S309: NO), or when the process at step S310 is performed, the arithmetic unit 220 determines whether the value of the measurement flag of the reaching time data is "1" (step S311). When it is determined that the value of the measurement flag of the reaching time data is "1" (step S311: YES), the arithmetic unit 220 sets the counter value CNTC of the reaching time data to +1 (step S312).

Moreover, when it is determined that the value of the measurement flag of the reaching time data is not "1" (step S311: NO), or when the processing at step S312 is performed, the arithmetic unit 220 determines whether the image displayed on the display screen 101S corresponds to in either one of the periods T4, T6, T8, T10, and T12 (step S313) or not.

When it is determined that the image displayed on the display screen 101S corresponds to either one of the periods T4, T6, T8, T10, and T12 (step S313: YES), the determining unit 218 determines whether a gaze point is present in the specified region AP (step S314). When it is determined that a gaze point is present in the specified region AP by the determining unit 218 (step S314: YES),the arithmetic unit 220 sets the counter value CNTA of the first presence-time data to +1, and sets the value of the measurement flag of the reaching time data to "0" (step S315). Furthermore, when it is determined that a gaze point is not present in the specified region AP by the determining unit 218 (step S314: NO), the arithmetic unit 220 sets the counter value CNTB of the second presence-time data to +1 (step S316).

When the process at step S315 or the process at step S316 is performed, when it is determine that the image displayed on the display screen 101S does not correspond to any one of the periods T4, T6, T8, T10, and T12 (step S313: NO), or when detection of position data at step S306 is failed (step S306: NO), the arithmetic unit 220 determines whether it has come to a time to finish reproduction of the image based on a detection result of the management timer (step S317). When the arithmetic unit 220 determines that it has not come to the time to finish reproduction of the image (step S317: NO), the processes at step S305 and later described above is repeated.

When the arithmetic unit 220 determines that it has come to the time to finish reproduction of the image (step S317: YES), the display control unit 202 stops reproduction of the image (step S318). After reproduction of the image is stopped, the evaluating unit 224 calculates the evaluation value ANS based on the region data, the presence time data, and the reaching time data that are acquired from a result of the processes described above (step S319), to calculate evaluation data based on the evaluation value ANS. Thereafter, the output control unit 226 outputs the evaluation data calculated by the evaluating unit 224 (step S320).

As described above, the evaluation device 100 according to the present embodiment includes: the image-data acquiring unit 206 that acquires image data of an eyeball of a subject; the gaze-point detecting unit 214 that detects position data of a gaze point of the subject based on the image data; the display control unit 202 that performs the display operation to display the plural objects M1 to M5 on the display screen 101S, and the non-display operation to hide the objects M1 to M5 in predetermined timing (times t1, t2, t3, t4, t5) after the display operation is started; the region setting unit 216 that sets the plural corresponding regions A1 to A5 that respectively correspond to the objects M1 to M5 on the display screen 101S; the determining unit 218 that determines, based on the position data of a gaze point, whether a gaze point is present in each of the corresponding regions A1 to A5 in the non-display period (periods T4, T6, T8, T10, T12) in which the non-display operation is performed, and outputs determination data; the arithmetic unit 220 that calculates, based on the determination data, respective pieces of region data indicating the corresponding regions A1 to A5 in which a gaze point is detected in the non-display period out of the corresponding regions A1 to A5; the evaluating unit 224 that calculates evaluation data of the subject based on the region data; and the output control unit 226 that outputs the evaluation data.

With this configuration, the region data indicating the corresponding regions A1 to A5 in which a gaze point of a subject has been detected in the non-display period is calculated, and evaluation data of the subject is calculated based on the region data. Therefore, the evaluation device 100 can evaluate a memory of the subject based on movement of a line of sight of the subject in the non-display period. Thus, the evaluation device 100 can perform evaluation of a subject with high accuracy.

Furthermore, in the evaluation device 100 according to the present embodiment, the arithmetic unit 220 calculates presence time data based on presence time in which a gaze point is present in the corresponding regions A1 to A5 in the non-display period based on determination data, and the evaluating unit 224 calculates evaluation data based on the region data and the presence time data. Thus, kinds of data used at calculating evaluation data increase and, therefore, a memory of a subject can be evaluated with higher accuracy.

Moreover, in the evaluation device 100 according to the present embodiment, the presence time data includes the first presence-time data that indicates the first presence time in which a gaze point is present in the specified region AP being a predetermined corresponding region out of the corresponding regions A1 to A5, and the second presence-time data indicating the second presence time in which a gaze point is present in the corresponding regions A1 to A5 that is not the specified region AP. Thus, kinds of data used at acquiring evaluation data increase and data becomes more precise. Therefore, a memory of a subject can be evaluated with higher accuracy.

Furthermore, in the evaluation device 100 according to the present embodiment, the display control unit 202 performs the display operation and the non-display operation repeatedly multiple times, and the arithmetic unit 220 calculates the first presence-time data and the second presence-time data throughout the periods T4, T6, T8, T10, and T12. Thus, in performing the non-display operation for multiple times, a memory of a subject can be evaluated comprehensively.

Moreover, in the evaluation device 100 according to the present embodiment, the arithmetic unit 220 calculates the reaching time data that indicates a time until a gaze point first reaches the specified region AP from a start time of the non-display period based on the determination data, and the evaluating unit 224 calculates evaluation data based on the region data, the presence time data, and the reaching time data. Thus, kinds of data used at acquiring evaluation data further increase and, therefore, a memory of a subject can be evaluated with higher accuracy.

Furthermore, in the evaluation device 100 according to the present embodiment, the display control unit 202 displays the range regions H1 to H5 indicating ranges of the respective corresponding regions A1 to A5 on the display screen 101S in the non-display period. Thus, it is possible to make it easy for a subject to bring an eyepoint to the corresponding regions A1 to A5.

### Second Embodiment

A second embodiment is described. In the following description, the same reference signs are given to the same or equivalent components as those in the embodiment described above, and explanation thereof is simplified or omitted. In the first embodiment, it has been described that the counter value CNTA indicating the first presence time (first presence-time data) and the counter value CNTB indicating the second presence time (second presence-time data) are total values throughout the respective periods T4, T6, T8, T10, and T12, but it is not limited thereto. In the present embodiment, a case in which a counter value indicating the first presence time (first presence-time data) and a counter value indicating the second presence time (second presence-time data) are calculated independently for each of the periods T4, T6, T8, T10, and T12 is described.

In each of the periods T4, T6, T8, T10, and T12, a counter provided in the arithmetic unit 220 counts the first presence-time data, the second presence-time data, and the reaching time data. For example, the counter value of the first presence time in the period T4 is referred to as CNTA1, and the counter value of the second presence time data is referred to as CNTB1. Moreover, the counter value of the first presence time in the period T6 is referred to as CNTA2, and the counter value of the second presence time data is referred to as CNTB2. Furthermore, the counter value of the first presence time in the period T8 is referred to as CNTA3, and the counter value of the second presence time data is referred to as CNTB3. Furthermore, the counter value of the first presence time in the period T10 is referred to as CNTA4, and the counter value of the second presence time data is referred to as CNTB4. Moreover, the counter value of the first presence time in the period T12 is referred to as CNTA5, and the counter value of the second presence time data is referred to as CNTB5.

In the present embodiment, an evaluation value to calculate evaluation data can be calculated for each of the periods T4, T6, T8, T10, and T12.

For example, when evaluation value is calculated by determining length of time in which a gaze point of the subject is present in the specified region AP, evaluation value can be calculated by determining whether a value of each of the counter values CNTA1 to CNTA5 is equal to or larger than a predetermined value. For example, when values of the counter values CNTA1 to CNTA 5 are equal to or larger than the predetermined value, it is determined that the subject has been looking at the specified regions AP, and a correctness evaluation value in each period is set to a correct answer value (for example, +1). Moreover, when values of the counter values CNTA1 to CNTA 5 are smaller than the predetermined value, it is determined that the subject has not been looking at the specified regions AP, and a correctness evaluation value in each period is set to an incorrect answer value (for example, 0). Furthermore, the evaluation value is calculated based on a total value (0, 1, 2, 3, 4, or 5) of the correctness evaluation values in the periods.

Moreover, for example, the evaluation value can be calculated by Equations (3) to (7) below.$ANS 11 = CNTA 1 / \left(CNTA1+CNTB1\right)$ $ANS 12 = CNTA 2 / \left(CNTA2+CNTB2\right)$ $ANS 13 = CNTA 3 / \left(CNTA3+CNTB3\right)$ $ANS 14 = CNTA 4 / \left(CNTA4+CNTB4\right)$ $ANS 15 = CNTA 5 / \left(CNTA5+CNTB5\right)$

In above Equations (1) to (7), specified-region gaze rates ANS11 to ANS15 in the respective periods T4, T6, T8, T10, and T12 are calculated. When values of the specified-region gaze rates ANS11 to ANS15 are equal to or larger than a predetermined value, it is determined that the subject has been looking at the specified regions AP, and the correctness evaluation value of each period is set to the correct answer value (for example, +1). Furthermore, values of the specified-region gaze rates ANS11 to ANS15 are smaller than the predetermined value, it is determined that the subject has not been looking at the specified regions AP, and the correctness evaluation value of each period is set to the incorrect answer value (for example, 0). Moreover, the evaluation value is calculated based on a total value (0, 1, 2, 3, 4, or 5) of the correctness evaluation values in the periods.

Furthermore, for example, the evaluation value can be calculated by Equations (8) to (12) below.$ANS 01 = ANS 11 \times K 11 + ANS 2 \times K 21$ $ANS 02 = ANS 12 \times K 12 + ANS 2 \times K 22$ $ANS 03 = ANS 13 \times K 13 + ANS 2 \times K 23$ $ANS 04 = ANS 14 \times K 14 + ANS 2 \times K 24$ $ANS 05 = ANS 15 \times K 15 + ANS 2 \times K 25$ (where ASN2 = K3 - CNTC)

In above Equations (8) to (12), period evaluation values ANS01 to ANS05 in the respective periods T4, T6, T8, T10, and T12 are calculated. When the period evaluation values ANS01 to ANS05 are equal to or larger than a predetermined value, it is determined that the subject has been looking at the specified regions AP, and the correctness evaluation value in each period is set to a correct answer value (for example, +1). Moreover, when the period evaluation values ANS01 to ANS05 are smaller than a predetermined value, it is determined that the subject has not been looking at the specified regions AP, and the correctness evaluation value in each period is set to an incorrect answer value (for example, 0). Furthermore, the evaluation value is calculated based on a total value (0, 1, 2, 3, 4, or 5) of the correctness evaluation values in the periods. The constants K11 to K15, K21 to K25 are constants for weighting.

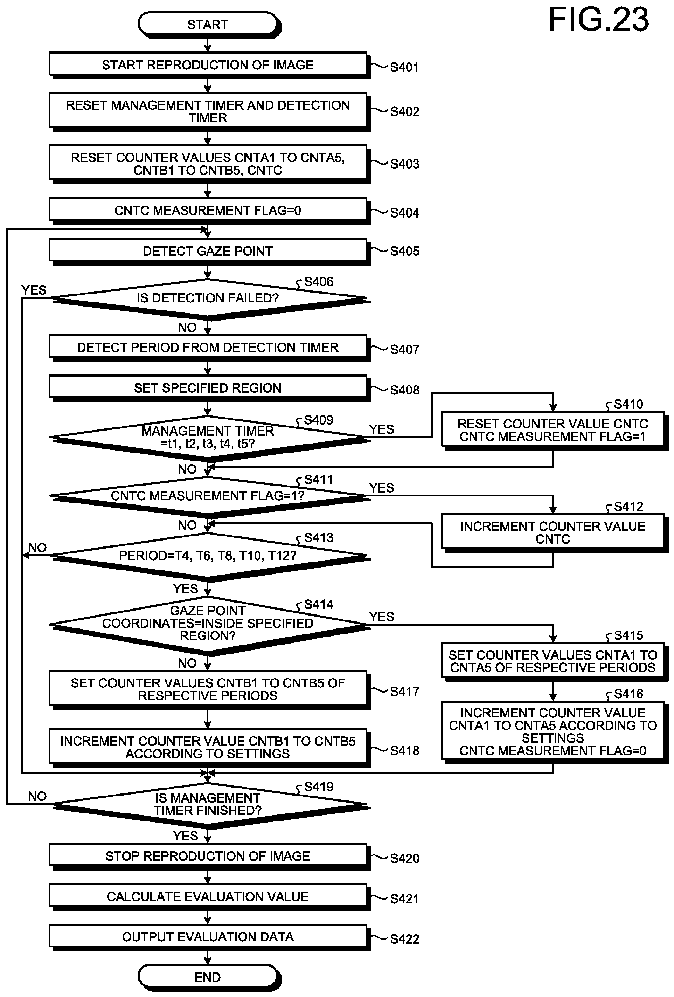

Next, an example of an evaluation method according to the second embodiment is described, referring to FIG. 23. FIG. 23 is a flowchart showing an example of the evaluation method according to the second embodiment. In the present embodiment, the display control unit 202 starts reproduction of an image (step S401). On the display screen 101S, images illustrated in FIG. 14 to FIG. 20 are sequentially displayed. In the following, each process at the step S402 to step S404 is similar to each process at step S302 to step S314 in the first embodiment.

When it is determined that a gaze point is present in the specified region AP by the determining unit 218 (step S414: YES), the arithmetic unit 220 sets the counter values CNTA1 to CNTA5 of the first presence-time data corresponding to the periods T4, T6, T8, T10, and T12 (step S415). The arithmetic unit 220 sets the set counter value (either one of CNTA1 to CNTA5) to +1, and sets the value of the measurement flag of the reaching time data to "0" (step S416). Furthermore, when it is determined that a gaze point is not present in the specified region AP by the determining unit 218, the arithmetic unit 220 sets the counter values CNTB1 to CNTB5 of the second presence-time data corresponding to the periods T4, T6, T8, T10, and T12 (step S417), and sets the set counter value (either one of CNTA1 to CNTA5) to +1 (step S418).

When process at step S416 or step S418 is performed, or when it is determined that an image displayed on the display screen 101S does not correspond to any of the periods T4, T6, T8, T10, and T12 (step S413: NO), or when detection of position data at step S406 is failed (step S406: NO), the arithmetic unit 220 determines whether it has come to the time to finish reproduction of the image based on a detection result of the management timer (step S419). When it is determined that it has not come to the time to finish reproduction of the image by the arithmetic unit 220 (step S419: NO), the process at step S405 and later described above is repeated.

When it is determined that it has come to the time to finish reproduction of the image by the arithmetic unit 220 (step S419: YES), the display control unit 202 stops reproduction of the image (step S420). After reproduction of the image is stopped, the evaluating unit 224 calculates the evaluation value ANS based on the region data, the presence time data, and the reaching time data acquired from a result of processes described above (step S421), and acquires evaluation data based on the evaluation value ANS. Thereafter, the output control unit 226 outputs the evaluation value acquired by the evaluating unit 224 (step S422).

As described above, according to the present embodiment, the first presence time (first presence-time data) and the second presence time (second presence-time data) are independently calculated for each of the periods T4, T6, T8, T10, and T12. Thus, kinds of data used at acquiring evaluation data increase and data becomes more precise. Therefore, a memory of a subject can be evaluated with higher accuracy.

The technical scope of the present invention is not limited to the embodiments described above, the invention is limited only by the scope of the appended claims.

### Reference Signs List

A0 INSTRUCTION REGION
A1 TO A5 CORRESPONDING REGION
AP SPECIFIED REGION
H1 TO H5 RANGE REGION
M1 TO M5 OBJECT
P PLOT POINT
165, 166 GAZE POINT
P0 INITIAL POSITION
T1 TO T13 PERIOD
ANS EVALUATION VALUE
ANS1, ANS11 TO ANS15 SPECIFIED-REGION GAZE RATE
ANS2 REACHING-TIME EVALUATION VALUE
CNTA, CNTB, CNTC, CNTA1 TO CNTA5, CNTB1 TO CNTB5 COUNTER VALUE
ANS01 TO ANS05 PERIOD EVALUATION VALUE
20 COMPUTER SYSTEM
20A ARITHMETIC PROCESSING DEVICE
20B STORAGE DEVICE
20C COMPUTER PROGRAM
30 INPUT/OUTPUT INTERFACE DEVICE
40 DRIVING CIRCUIT
50 OUTPUT DEVICE
60 INPUT DEVICE
70 VOICE OUTPUT DEVICE
100 EYE TRACKING DEVICE, EVALUATION DEVICE
101 DISPLAY DEVICE
101S DISPLAY SCREEN
102 STEREO CAMERA DEVICE
102A FIRST CAMERA
102B SECOND CAMERA
103 ILLUMINATION DEVICE
103A FIRST LIGHT SOURCE
103B SECOND LIGHT SOURCE
103C LIGHT SOURCE
103V VIRTUAL LIGHT SOURCE
109 CORNEAL CURVATURE RADIUS
110, 110H CORNEAL CURVATURE CENTER
111 EYEBALL
112 PUPIL
112C PUPIL CENTER
113 CORNEAL REFLECTION IMAGE
113C, 121, 122, 124 CORNEAL REFLECTION CENTER
123, 131, 132, 173 STRAIGHT LINE
126 DISTANCE
130 TARGET POSITION
177, 178 LINE OF SIGHT
202 DISPLAY CONTROL UNIT
204 LIGHT-SOURCE CONTROL UNIT
206 IMAGE-DATA ACQUIRING UNIT
208 INPUT-DATA ACQUIRING UNIT
210 POSITION DETECTING UNIT
212 CURVATURE-CENTER CALCULATING UNIT
214 GAZE-POINT DETECTING UNIT
216 REGION SETTING UNIT
218 DETERMINING UNIT
220 ARITHMETIC UNIT
222 STORAGE UNIT
224 EVALUATING UNIT
226 OUTPUT CONTROL UNIT
302 INPUT/OUTPUT UNIT
402 DISPLAY-DEVICE DRIVING UNIT
404A FIRST-CAMERA INPUT/OUTPUT UNIT
404B SECOND-CAMERA INPUT/OUTPUT UNIT
406 LIGHT-SOURCE DRIVING UNIT

## Claims

1. An evaluation device (100) comprising:
an image-data acquiring unit (206) configured to acquire image data of an eyeball (111) of a subject;
a gaze-point detecting unit (214) configured to detect position data of a gaze point (165) of the subject based on the image data;
a display control unit (202) configured to perform a display operation to display a plurality of objects (M1 to M5) on a display screen (101S) in a separated manner from one another, and a non-display operation to delete a display of the plurality of objects (M1 to M5) from the display screen (101S) in predetermined timing after the display operation is started;
a region setting unit (216) configured to set a plurality of corresponding regions (A1 to A5) that correspond to the objects (M1 to M5), respectively, on the display screen (101S);
a determining unit (218) configured to determine, based on the position data of the gaze point (165), whether the gaze point (165) is present in the corresponding region (A1 to A5) in a non-display period (T4, T6, T8, T10, T12) in which the non-display operation is performed;
an arithmetic unit (220) configured to calculate, based on determination data, region data that indicates the corresponding region (A1 to A5) in which the gaze point (165) is detected in the non-display period (T4, T6, T8, T10, T12) out of the corresponding regions (A1 to A5); and
an evaluating unit (224) configured to calculate evaluation data of the subject based on the region data, wherein
the display control unit (202) is configured to display an instruction instructing the subject to look at a specific region, which is one of the corresponding region corresponding to a specific object out of the objects (M1 to M5) to be look at by the subject in the non-display period (T4, T6, T8, T10, T12),
the region setting unit is configured to set the specific region as a specified region,
the arithmetic unit (220) is configured to further calculate, based on the determination data, presence time data which is based on presence time in which the gaze point (165) is present in the corresponding region (A1 to A5) in the non-display period (T4, T6, T8, T10, T12), the presence time data including first presence-time data that indicates first presence time in which the gaze point (165) is present in the specified region in the non-display period (T4, T6, T8, T10, T12), and second presence-time data that indicates second presence time in which the gaze point (165) is present in the corresponding region (A1 to A5) that is not the specified region in the non-display period (T4, T6, T8, T10, T12),
the display control unit (202) is configured to perform the display operation and the non-display operation repeatedly multiple times, and
the evaluating unit (224) is configured to calculate the evaluation data based on the region data and the presence time data, wherein
the arithmetic unit (220) is configured to calculate the first presence-time data and the second presence-time data throughout the non-display periods (T4, T6, T8, T10, T12) of the non-display operation, or
the arithmetic unit (220) is configured to calculate the first presence-time data and the second presence-time data for each non-display period (T4, T6, T8, T10, T12) of the non-display operation.

2. The evaluation device (100) according to claim 1, wherein
the arithmetic unit (220) is configured to calculate reaching time data that indicates a time until the gaze point (165) first reaches the specified region from a start time (t1) of the non-display operation, based on the determination data, and
the evaluating unit (224) is configured to calculate the evaluation data based on the region data, the presence time data, and the reaching time data.

3. The evaluation device (100) according to claims 1 or 2, wherein
the display control unit (202) is configured to display a range region (H1 to H5) that indicates ranges of the respective corresponding regions (A1 to A5) on the display screen (101S) in the non-display operation.

4. The evaluation device (100) according to claims 1 to 3, further comprising
an output control unit (226) configured to display plot points (P) that indicate the position data of the gaze point of the subject on the display screen (101S)

5. The evaluation device (100) according to claims 1 to 4, wherein
the arithmetic unit (220) has a counter configured to count the first presence-time data and the second presence-time data,
the evaluating unit (224) is configured to calculate the evaluation data by determining whether a specified-region gaze rate (ANS1) is equal to or larger than a predetermined value for evaluating whether the subject is likely to have cranial nerve disease or disorder, where the specified-region gaze rate (ANS1) indicates a ratio of a counter value of the first presence time (CNTA) to a sum of the counter value of the first presence time (CNTA) and a counter value of the second presence time (CNTB).

6. An evaluation method performed by an evaluation device (100) comprising:
acquiring by an an image-data acquiring unit (206) image data of an eyeball (111) of a subject;
detecting by a gaze-point detecting unit (214) position data of a gaze point (165) of the subject based on the image data;
performing by a display control unit (202) a display operation to display a plurality of objects (M1 to M5) on a display screen (101S) in a separated manner from one another, and a non-display operation to delete a display of the plurality of objects (M1 to M5) from the display screen (101S) in predetermined timing after the display operation is started;
setting by a region setting unit (216) a plurality of corresponding regions (A1 to A5) that correspond to the objects, respectively, on the display screen (101S);
determining, by a determining unit (218), based on the position data of the gaze point (165), whether the gaze point (165) is present in the corresponding region (A1 to A5) in a non-display period (T4, T6, T8, T10, T12) in which the non-display operation is performed;
calculating, by an an arithmetic unit (220), based on determination data, region data that indicates the corresponding region (A1 to A5) in which the gaze point (165) is detected in the non-display period (T4, T6, T8, T10, T12) out of the corresponding regions (A1 to A5);
calculating by an evaluating unit (224) evaluation data of the subject based on the region data; and
displaying by the display control unit (202) an instruction instructing the subject to look at a specific region, which is one of the corresponding region corresponding to a specific object out of the objects (M1 to M5) to be look at by the subject in the non-display period, wherein
the setting further includes setting the specific region as a specified region,
the calculating the region data further includes calculating, based on the determination data, presence time data which is based on presence time in which the gaze point (165) is present in the corresponding region (A1 to A5) in the non-display period (T4, T6, T8, T10, T12), the presence time data including first presence-time data that indicates first presence time in which the gaze point (165) is present in the specified region in the non-display period (T4, T6, T8, T10, T12), and second presence-time data that indicates second presence time in which the gaze point (165) is present in the corresponding region (A1 to A5) that is not the specified region in the non-display period (T4, T6, T8, T10, T12),
the performing includes performing the display operation and the non-display operation repeatedly multiple times, and
the calculating the evaluating data includes calculating the evaluation data based on the region data and the presence time data, wherein
the calculating the region data includes calculating the first presence-time data and the second presence-time data throughout the non-display periods (T4, T6, T8, T10, T12) of the non-display operation, or calculating the first presence-time data and the second presence-time data for each non-display period (T4, T6, T8, T10, T12) of the non-display operation.

7. An evaluation program that causes the evaluation device of claim 1 to execute:
a process of acquiring image data of an eyeball (111) of a subject;
a process of detecting position data of a gaze point (165)of the subject based on the image data;
a process of performing a display operation to display a plurality of objects (M1 to M5) on a display screen (101S) in a separated manner from one another, and a non-display operation to delete a display of the plurality of objects (M1 to M5) from the display screen (101S) in predetermined timing after the display operation is started;
a process of setting a plurality of corresponding regions (A1 to A5) that correspond to the objects (M1 to M5), respectively, on the display screen (101S);
a process of determining, based on the position data of the gaze point (165), whether the gaze point (165) is present in the corresponding region (A1 to A5) in a non-display period (T4, T6, T8, T10, T12) in which the non-display operation is performed;
a process of calculating, based on determination data, region data that indicates the corresponding region (A1 to A5) in which the gaze point (165) is detected in the non-display period (T4, T6, T8, T10, T12) out of the corresponding regions (A1 to A5);
a process of calculating evaluation data of the subject based on the region data; and
a process of displaying an instruction instructing the subject to look at a specific region, which is one of the corresponding region corresponding to a specific object out of the objects (M1 to M5) to be look at by the subject in the non-display period, wherein
the process of setting further includes setting the specified region,
the process of calculating the region data further includes calculating, based on the determination data, presence time data which is based on presence time in which the gaze point (165) is present in the corresponding region (A1 to A5) in the non-display period (T4, T6, T8, T10, T12), the presence time data including first presence-time data that indicates first presence time in which the gaze point (165) is present in the specified region in the non-display period (T4, T6, T8, T10, T12), and second presence-time data that indicates second presence time in which the gaze point (165) is present in the corresponding region (A1 to A5) that is not the specified region in the non-display period (T4, T6, T8, T10, T12),
the process of performing includes performing the display operation and the non-display operation repeatedly multiple times, and
the process of calculating the evaluating data includes calculating the evaluation data based on the region data and the presence time data, wherein
the calculating the region data includes calculating the first presence-time data and the second presence-time data throughout the non-display periods (T4, T6, T8, T10, T12) of the non-display operation, or calculating the first presence-time data and the second presence-time data for each non-display period (T4, T6, T8, T10, T12) of the non-display operation.

## Patentansprüche

1. Ein Bewertungsgerät (100), aufweisend:
eine Bilddaten-Erhaltungseinheit (206), die konfiguriert ist zum Erhalten von Bilddaten eines Augapfels (111) einer Person,
eine Blickpunkt-Erfassungseinheit (214), die konfiguriert ist zum Erfassen von Positionsdaten eines Blickpunkts (165) der Person basierend auf den Bilddaten,
eine Anzeigesteuereinheit (202), die konfiguriert ist zum Durchführen einer Anzeigeoperation für das Anzeigen einer Vielzahl von Objekten (M1 bis M5) auf einem Anzeigebildschirm (101S) getrennt voneinander sowie einer nicht-Anzeigeoperation zum Löschen der Anzeige der Vielzahl von Objekten (M1 bis M5) von dem Anzeigebildschirm (101S) in einem vorbestimmten Timing nach dem Start der Anzeigeoperation,
eine Bereichssetzeinheit (216), die konfiguriert ist zum Setzen einer Vielzahl von entsprechenden Bereichen (A1 bis A5), die jeweils den Objekten (M1 bis M5) auf dem Anzeigebildschirm (101S) entsprechen,
eine Bestimmungseinheit (218), die konfiguriert ist zum Bestimmen, basierend auf den Positionsdaten des Blickpunkts (165), ob der Blickpunkt (165) in dem entsprechenden Bereich (A1 bis A5) in einer nicht-Anzeigeperiode (T4, T6, T8, T10, T12), in dem die nicht-Anzeigeoperation durchgeführt wird, präsent ist,
eine arithmetische Einheit (220), die konfiguriert ist zum Berechnen, basierend auf Bestimmungsdaten, von Bereichsdaten, die den entsprechenden Bereich (A1 bis A5), in dem der Blickpunkt (165) in der nicht-Anzeigeperiode (T4, T6, T8, T10, T12) erfasst wird, innerhalb der entsprechenden Bereiche (A1 bis A5) angeben, und
eine Bewertungseinheit (224), die konfiguriert ist zum Berechnen von Bewertungsdaten der Person basierend auf den Bereichsdaten, wobei:
die Anzeigesteuereinheit (202) konfiguriert ist zum Anzeigen eines Befehls, der die Person anweist, auf einen spezifischen Bereich zu blicken, der einer der entsprechenden Bereiche in Entsprechung zu einem spezifischen Objekt innerhalb der Objekte (M1 bis M5), auf das die Person in der nicht-Anzeigeperiode (T4, T6, T8, T10, T12) blicken soll, ist,
die Bereichssetzeinheit konfiguriert ist zum Setzen des spezifischen Bereichs als eines spezifizierten Bereichs,
die arithmetische Einheit (220) weiterhin konfiguriert ist zum Berechnen, basierend auf den Bestimmungsdaten, von Präsenzzeitdaten, die auf einer Präsenzzeit, in welcher der Blickpunkt (165) in dem entsprechenden Bereich (A1 bis A5) in der nicht-Anzeigeperiode (T4, T6, T8, T10, T12) präsent ist, basieren, wobei die Präsenzzeitdaten umfassen: erste Präsenzzeitdaten, die eine erste Präsenzzeit angeben, in welcher der Blickpunkt (165) in dem spezifizierten Bereich in der nicht-Anzeigeperiode (T4, T6, T8, T10, T12) präsent ist, und zweite Präsenzzeitdaten, die eine zweite Präsenzzeit angeben, in welcher der Blickpunkt (165) in dem entsprechenden Bereich (A1 bis A5), der nicht der spezifizierte Bereich in der nicht-Anzeigeperiode (T4, T6, T8, T10, T12) ist, präsent ist,
die Anzeigesteuereinheit (202) konfiguriert ist zum wiederholten und mehrfachen Durchführen der Anzeigeoperation und der nicht-Anzeigeoperation, und
die Bewertungseinheit (224) konfiguriert ist zum Berechnen der Bewertungsdaten basierend auf den Bereichsdaten und den Präsenzzeitdaten, wobei:
die arithmetische Einheit (220) konfiguriert ist zum Berechnen der ersten Präsenzzeitdaten und der zweiten Präsenzzeitdaten über die nicht-Anzeigeperioden (T4, T6, T8, T10, T12) der nicht-Anzeigeoperation hinweg, oder
die arithmetische Einheit (220) konfiguriert ist zum Berechnen der ersten Präsenzzeitdaten und der zweiten Präsenzzeitdaten für jede nicht-Anzeigeperiode (T4, T6, T8, T10, T12) der nicht-Anzeigeoperation.

2. Bewertungsgerät (100) nach Anspruch 1, wobei:
die arithmetische Einheit (220) konfiguriert ist zum Berechnen von Erreichungszeitdaten, die eine Zeit bis der Blickpunkt (165) zuerst den spezifizierten Bereich von einer Startzeit (t1) der nicht-Anzeigeoperation erreicht, basierend auf den Bestimmungsdaten angibt, und
die Bewertungseinheit (224) konfiguriert ist zum Berechnen der Bewertungsdaten basierend auf den Bereichsdaten, den Präsenzzeitdaten und den Erreichungszeitdaten.

3. Bewertungsgerät (100) nach Anspruch 1 oder 2, wobei:
die Anzeigesteuereinheit (202) konfiguriert ist zum Anzeigen eines Erstreckungsbereichs (H1 bis H5), der Erstreckungen der jeweiligen entsprechenden Bereiche (A1 bis A5) auf dem Anzeigebildschirm (101S) in der nicht-Anzeigeoperation angibt.

4. Bewertungsgerät (100) nach einem der Ansprüche 1 bis 3, das weiterhin aufweist:
eine Ausgabesteuereinheit (226), die konfiguriert ist zum Anzeigen von Plotpunkten (P), die die Positionsdaten des Blickpunkts der Person auf dem Anzeigebildschirm (101S) angeben.

5. Bewertungsgerät (100) nach einem der Ansprüche 1 bis 4, wobei:
die arithmetische Einheit (220) einen Zähler aufweist, der konfiguriert ist zum Zählen der ersten Präsenzzeitdaten und der zweiten Präsenzzeitdaten,
die Bewertungseinheit (224) konfiguriert ist zum Berechnen der Bewertungsdaten durch das Bestimmen, ob eine Spezifizierter-Bereich-Blickrate (ANS1) gleich oder größer als ein vorbestimmter Wert ist, um zu bewerten, ob eine Wahrscheinlichkeit dafür besteht, dass die Person eine Hirnnervenerkrankung oder -störung aufweist, wobei die Spezifizierter-Bereich-Blickrate (ANS1) ein Verhältnis eines Zählerwerts der ersten Präsenzzeit (CNTA) zu einer Summe aus dem Zählerwert der ersten Präsenzzeit (CNTA) und einem Zählerwert der zweiten Präsenzzeit (CNTB) angibt.

6. Bewertungsverfahren, das durch ein Bewertungsgerät (100) durchgeführt wird, aufweisend:
Erhalten, durch eine Bilddaten-Erhaltungseinheit (206), von Bilddaten eines Augapfels (111) einer Person,
Erfassen, durch eine Blickpunkt-Erfassungseinheit (214), von Positionsdaten eines Blickpunkts (165) der Person basierend auf den Bilddaten,
Durchführen, durch eine Anzeigesteuereinheit (202), einer Anzeigeoperation für das Anzeigen einer Vielzahl von Objekten (M1 bis M5) auf einem Anzeigebildschirm (101S) getrennt voneinander sowie einer nicht-Anzeigeoperation zum Löschen der Anzeige der Vielzahl von Objekten (M1 bis M5) von dem Anzeigebildschirm (101S) in einem vorbestimmten Timing nach dem Start der Anzeigeoperation,
Setzen, durch eine Bereichssetzeinheit (216), einer Vielzahl von entsprechenden Bereichen (A1 bis A5), die jeweils den Objekten auf dem Anzeigebildschirm (101S) entsprechen,
Bestimmen, durch eine Bestimmungseinheit (218) und basierend auf den Positionsdaten des Blickpunkts (165), ob der Blickpunkt (165) in dem entsprechenden Bereich (A1 bis A5) in einer nicht-Anzeigeperiode (T4, T6, T8, T10, T12), in dem die nicht-Anzeigeoperation durchgeführt wird, präsent ist,
Berechnen, durch eine arithmetische Einheit (220) und basierend auf Bestimmungsdaten, von Bereichsdaten, die den entsprechenden Bereich (A1 bis A5), in dem der Blickpunkt (165) in der nicht-Anzeigeperiode (T4, T6, T8, T10, T12) erfasst wird, innerhalb der entsprechenden Bereiche (A1 bis A5) angeben, und
Berechnen, durch eine Bewertungseinheit (224), von Bewertungsdaten der Person basierend auf den Bereichsdaten, und
Anzeigen, durch die Anzeigesteuereinheit (202), eines Befehls, der die Person anweist, auf einen spezifischen Bereich zu blicken, der einer der entsprechenden Bereiche in Entsprechung zu einem spezifischen Objekt innerhalb der Objekte (M1 bis M5), auf das die Person in der nicht-Anzeigeperiode blicken soll, ist, wobei:
das Setzen weiterhin das Setzen des spezifischen Bereichs als eines spezifizierten Bereichs aufweist,
das Berechnen der Bereichsdaten weiterhin das Berechnen, basierend auf den Bestimmungsdaten, von Präsenzzeitdaten, die auf einer Präsenzzeit, in welcher der Blickpunkt (165) in dem entsprechenden Bereich (A1 bis A5) in der nicht-Anzeigeperiode (T4, T6, T8, T10, T12) präsent ist, basieren, wobei die Präsenzzeitdaten umfassen: erste Präsenzzeitdaten, die eine erste Präsenzzeit angeben, in welcher der Blickpunkt (165) in dem spezifizierten Bereich in der nicht-Anzeigeperiode (T4, T6, T8, T10, T12) präsent ist, und zweite Präsenzzeitdaten, die eine zweite Präsenzzeit angeben, in welcher der Blickpunkt (165) in dem entsprechenden Bereich (A1 bis A5), der nicht der spezifizierte Bereich in der nicht-Anzeigeperiode (T4, T6, T8, T10, T12) ist, präsent ist,
das Durchführen das wiederholte und mehrfache Durchführen der Anzeigeoperation und der nicht-Anzeigeoperation umfasst, und
das Berechnen der Bewertungsdaten das Berechnen der Bewertungsdaten basierend auf den Bereichsdaten und den Präsenzzeitdaten umfasst, wobei:
das Berechnen der Bereichsdaten das Berechnen der ersten Präsenzzeitdaten und der zweiten Präsenzzeitdaten über die nicht-Anzeigeperioden (T4, T6, T8, T10, T12) der nicht-Anzeigeoperation hinweg oder das Berechnen der ersten Präsenzzeitdaten und der zweiten Präsenzzeitdaten für jede nicht-Anzeigeperiode (T4, T6, T8, T10, T12) der nicht-Anzeigeoperation umfasst.

7. Ein Bewertungsprogramm, das das Bewertungsgerät gemäß Anspruch 1 veranlasst zum Ausführen:
eines Prozesses zum Erhalten von Bilddaten eines Augapfels (111) einer Person,
eines Prozesses zum Erfassen von Positionsdaten eines Blickpunkts (165) der Person basierend auf den Bilddaten,
eines Prozesses zum Durchführen einer Anzeigeoperation für das Anzeigen einer Vielzahl von Objekten (M1 bis M5) auf einem Anzeigebildschirm (101S) getrennt voneinander sowie einer nicht-Anzeigeoperation zum Löschen der Anzeige der Vielzahl von Objekten (M1 bis M5) von dem Anzeigebildschirm (101S) in einem vorbestimmten Timing nach dem Start der Anzeigeoperation,
eines Prozesses zum Setzen einer Vielzahl von entsprechenden Bereichen (A1 bis A5), die jeweils den Objekten (M1 bis M5) auf dem Anzeigebildschirm (101S) entsprechen,
eines Prozesses zum Bestimmen, basierend auf den Positionsdaten des Blickpunkts (165), ob der Blickpunkt (165) in dem entsprechenden Bereich (A1 bis A5) in einer nicht-Anzeigeperiode (T4, T6, T8, T10, T12), in dem die nicht-Anzeigeoperation durchgeführt wird, präsent ist,
eines Prozesses zum Berechnen, basierend auf Bestimmungsdaten, von Bereichsdaten, die den entsprechenden Bereich (A1 bis A5), in dem der Blickpunkt (165) in der nicht-Anzeigeperiode (T4, T6, T8, T10, T12) erfasst wird, innerhalb der entsprechenden Bereiche (A1 bis A5) angeben, und
eines Prozesses zum Berechnen von Bewertungsdaten der Person basierend auf den Bereichsdaten, und
eines Prozesses zum Anzeigen eines Befehls, der die Person anweist, auf einen spezifischen Bereich zu blicken, der einer der entsprechenden Bereiche in Entsprechung zu einem spezifischen Objekt innerhalb der Objekte (M1 bis M5), auf das die Person in der nicht-Anzeigeperiode blicken soll, ist, wobei:
der Prozess zum Setzen weiterhin das Setzen des spezifizierten Bereichs aufweist,
der Prozess zum Berechnen der Bereichsdaten weiterhin das Berechnen, basierend auf den Bestimmungsdaten, von Präsenzzeitdaten, die auf einer Präsenzzeit, in welcher der Blickpunkt (165) in dem entsprechenden Bereich (A1 bis A5) in der nicht-Anzeigeperiode (T4, T6, T8, T10, T12) präsent ist, basieren, wobei die Präsenzzeitdaten umfassen: erste Präsenzzeitdaten, die eine erste Präsenzzeit angeben, in welcher der Blickpunkt (165) in dem spezifizierten Bereich in der nicht-Anzeigeperiode (T4, T6, T8, T10, T12) präsent ist, und zweite Präsenzzeitdaten, die eine zweite Präsenzzeit angeben, in welcher der Blickpunkt (165) in dem entsprechenden Bereich (A1 bis A5), der nicht der spezifizierte Bereich in der nicht-Anzeigeperiode (T4, T6, T8, T10, T12) ist, präsent ist,
der Prozess zum Durchführen das wiederholte und mehrfache Durchführen der Anzeigeoperation und der nicht-Anzeigeoperation umfasst, und
der Prozess zum Berechnen der Bewertungsdaten das Berechnen der Bewertungsdaten basierend auf den Bereichsdaten und den Präsenzzeitdaten umfasst, wobei:
das Berechnen der Bereichsdaten das Berechnen der ersten Präsenzzeitdaten und der zweiten Präsenzzeitdaten über die nicht-Anzeigeperioden (T4, T6, T8, T10, T12) der nicht-Anzeigeoperation hinweg oder das Berechnen der ersten Präsenzzeitdaten und der zweiten Präsenzzeitdaten für jede nicht-Anzeigeperiode (T4, T6, T8, T10, T12) der nicht-Anzeigeoperation umfasst.

## Revendications

1. Dispositif d'évaluation (100), comprenant :
une unité d'acquisition de données d'image (206) configurée pour acquérir des données d'image d'un globe oculaire (111) d'un sujet ;
une unité de détection du point de regard (214) configurée pour détecter des données de position d'un point de regard (165) du sujet sur la base des données d'image ;
une unité de commande d'affichage (202) configurée pour effectuer une opération d'affichage pour afficher une pluralité d'objets (M1 à M5) sur un écran d'affichage (101S) séparément les uns des autres, et une opération de non-affichage pour supprimer un affichage de la pluralité d'objets (M1 à M5) sur l'écran d'affichage (101S) dans un délai prédéterminé après le démarrage de l'opération d'affichage ;
une unité de définition de région (216) configurée pour définir une pluralité de régions correspondantes (A1 à A5) qui correspondent respectivement aux objets (M1 à M5) sur l'écran d'affichage (101S) ;
une unité de détermination (218) configurée pour déterminer, sur la base des données de position du point de regard (165), si le point de regard (165) est présent dans la région correspondante (A1 à A5) pendant une période de non-affichage (T4, T6, T8, T10, T12) pendant laquelle l'opération de non-affichage est effectuée ;
une unité arithmétique (220) configurée pour calculer, sur la base de données de détermination, des données de région qui indiquent la région correspondante (A1 à A5) dans laquelle le point de regard (165) est détecté pendant la période de non-affichage (T4, T6, T8, T10, T12), parmi les régions correspondantes (A1 à A5) ; et
une unité d'évaluation (224) configurée pour calculer des données d'évaluation du sujet sur la base des données de régions, dans lequel
l'unité de commande d'affichage (202) est configurée pour afficher une instruction demandant au sujet de regarder une région particulière qui est celle de la région correspondante qui correspond à un objet particulier parmi les objets (M1 à M5) qui doit être regardé par le sujet pendant la période de non-affichage (T4, T6, T8, T10, T12),
l'unité de définition de région est configurée pour définir la région particulière comme étant une région spécifiée
l'unité arithmétique (220) est configurée pour calculer en outre, sur la base des données de détermination, des données de temps de présence qui sont basées sur le temps de présence pendant lequel le point de regard (165) est présent dans la région correspondante (A1 à A5) pendant la période de non-affichage (T4, T6, T8, T10, T12), les données de temps de présence comportant des premières données de temps de présence qui indiquent un premier temps de présence pendant lequel le point de regard (165) est présent dans la région spécifiée pendant la période de non-affichage (T4, T6, T8, T10, T12), et des deuxièmes données de temps de présence qui indiquent un deuxième temps de présence pendant lequel le point de regard (165) est présent dans la région correspondante (A1 à A5) qui n'est pas la région spécifiée pendant la période de non-affichage (T4, T6, T8, T10, T12),
l'unité de commande d'affichage (202) est configurée pour effectuer l'opération d'affichage et l'opération de non-affichage de façon répétitive à plusieurs reprises, et
l'unité d'évaluation (224) est configurée pour calculer les données d'évaluation sur la base des données de région et des données de temps de présence, dans lequel
l'unité arithmétique (220) est configurée pour calculer les premières données de temps de présence et les deuxièmes données de temps de présence tout au long des périodes de non-affichage (T4, T6, T8, T10, T12) de l'opération de non-affichage, ou
l'unité arithmétique (220) est configurée pour calculer les premières données de temps de présence et les deuxièmes données de temps de présence pour chaque période de non-affichage (T4, T6, T8, T10, T12) de l'opération de non-affichage.

2. Dispositif d'évaluation (100) selon la revendication 1, dans lequel
l'unité arithmétique (220) est configurée pour calculer des données de temps d'atteinte qui indiquent un temps jusqu'à ce que le point de regard (165) atteigne pour la première fois la région spécifiée à partir d'un temps de début (t1) de l'opération de non-affichage, sur la base des données de détermination, et
l'unité d'évaluation (224) est configurée pour calculer les données d'évaluation sur la base des données de région, des données de temps de présence et des données de temps d'atteinte.

3. Dispositif d'évaluation (100) selon l'une quelconque des revendications 1 ou 2, dans lequel
l'unité de commande d'affichage (202) est configurée pour afficher une région de plage (H1 à H5) qui indique des plages des régions correspondantes respectives (A1 à A5) sur l'écran d'affichage (101S) en mode non-affichage.

4. Dispositif d'évaluation (100) selon l'une quelconque des revendications 1 à 3, comprenant en outre :
une unité de commande de sortie (226) configurée pour afficher des points de traceur (P) qui indiquent les données de position du point de regard du sujet sur l'écran d'affichage (101S).

5. Dispositif d'évaluation (100) selon l'une quelconque des revendications 1 à 4, dans lequel
l'unité arithmétique (220) a un compteur configuré pour compter les premières données de temps de présence et les deuxièmes données de temps de présence,
l'unité d'évaluation (224) est configurée pour calculer les données d'évaluation en déterminant si un taux de regard de la région spécifiée (ANS1) est égal ou supérieur à une valeur prédéterminée pour évaluer si le sujet est susceptible d'avoir une maladie ou un trouble nerveux cérébral, où le taux de regard de la région spécifiée (ANS1) indique un rapport d'une valeur de compteur du premier temps de présence (CNTA) sur une somme de la valeur de compteur du premier temps de présence (CNTA) et d'une valeur de compteur du deuxième temps de présence (CNTB).

6. Procédé d'évaluation effectué par un dispositif d'évaluation (100), comprenant :
l'acquisition, par une unité d'acquisition de données d'image (206), de données d'image d'un globe oculaire (111) d'un sujet ;
la détection, par une unité de détection du point de regard (214), de données de position d'un point de regard (165) du sujet sur la base des données d'image ;
l'exécution, par une unité de commande d'affichage (202), d'une opération d'affichage pour afficher une pluralité d'objets (M1 à M5) sur un écran d'affichage (101S) séparément les uns des autres, et une opération de non-affichage pour supprimer un affichage de la pluralité d'objets (M1 à M5) sur l'écran d'affichage (101S) dans un délai prédéterminé après le démarrage de l'opération d'affichage ;
la définition, par une unité de définition de région (216), d'une pluralité de régions correspondantes (A1 à A5) qui correspondent respectivement aux objets sur l'écran d'affichage (101S) ;
la détermination, par une unité de détermination (218), sur la base des données de position du point de regard (165), de si le point de regard (165) est présent dans la région correspondante (A1 à A5) pendant une période de non-affichage (T4, T6, T8, T10, T12) pendant laquelle l'opération de non-affichage est effectuée ;
le calcul, par une unité arithmétique (220), sur la base de données de détermination, de données de région qui indiquent la région correspondante (A1 à A5) dans laquelle le point de regard (165) est détecté pendant la période de non-affichage (T4, T6, T8, T10, T12), parmi les régions correspondantes (A1 à A5) ;
le calcul, par une unité d'évaluation (224), de données d'évaluation du sujet sur la base des données de régions ; et
l'affichage, par l'unité de commande d'affichage (202), d'une instruction demandant au sujet de regarder une région particulière qui est celle de la région correspondante qui correspond à un objet particulier parmi les objets (M1 à M5) qui doit être regardé par le sujet pendant la période de non-affichage,
la définition comporte en outre la définition de la région particulière comme étant une région spécifiée,
le calcul des données de région comporte en outre le calcul, sur la base des données de détermination, de données de temps de présence qui sont basées sur le temps de présence pendant lequel le point de regard (165) est présent dans la région correspondante (A1 à A5) pendant la période de non-affichage (T4, T6, T8, T10, T12), les données de temps de présence comportant des premières données de temps de présence qui indiquent un premier temps de présence pendant lequel le point de regard (165) est présent dans la région spécifiée pendant la période de non-affichage (T4, T6, T8, T10, T12), et des deuxièmes données de temps de présence qui indiquent un deuxième temps de présence pendant lequel le point de regard (165) est présent dans la région correspondante (A1 à A5) qui n'est pas la région spécifiée pendant la période de non-affichage (T4, T6, T8, T10, T12),
l'exécution comporte l'opération d'affichage et l'opération de non-affichage de façon répétitive à plusieurs reprises, et
le calcul des données d'évaluation comporte le calcul des données d'évaluation sur la base des données de région et des données de temps de présence, dans lequel
le calcul des données d'évaluation comporte le calcul des premières données de temps de présence et des deuxièmes données de temps de présence tout au long des périodes de non-affichage (T4, T6, T8, T10, T12) de l'opération de non-affichage, ou le calcul des premières données de temps de présence et des deuxièmes données de temps de présence pour chaque période de non-affichage (T4, T6, T8, T10, T12) de l'opération de non-affichage.

7. Programme d'évaluation qui amène le dispositif d'évaluation selon la revendication 1 à exécuter :
un processus d'acquisition de données d'image d'un globe oculaire (111) d'un sujet ;
un processus de détection de données de position d'un point de regard (165) du sujet sur la base des données d'image ;
un processus d'exécution d'une opération d'affichage pour afficher une pluralité d'objets (M1 à M5) sur un écran d'affichage (101S) séparément les uns des autres, et une opération de non-affichage pour supprimer un affichage de la pluralité d'objets (M1 à M5) sur l'écran d'affichage (101S) dans un délai prédéterminé après le démarrage de l'opération d'affichage ;
un processus de définition d'une pluralité de régions correspondantes (A1 à A5) qui correspondent respectivement aux objets (M1 à M5) sur l'écran d'affichage (101S) ;
un processus de détermination, sur la base des données de position du point de regard (165), de si le point de regard (165) est présent dans la région correspondante (A1 à A5) pendant une période de non-affichage (T4, T6, T8, T10, T12) pendant laquelle l'opération de non-affichage est effectuée ;
un processus de calcul, sur la base de données de détermination, de données de région qui indiquent la région correspondante (A1 à A5) dans laquelle le point de regard (165) est détecté pendant la période de non-affichage (T4, T6, T8, T10, T12), parmi les régions correspondantes (A1 à A5) ;
un processus de calcul de données d'évaluation du sujet sur la base des données de régions ; et
un processus d'affichage d'une instruction demandant au sujet de regarder une région particulière qui est celle de la région correspondante qui correspond à un objet particulier parmi les objets (M1 à M5) qui doit être regardé par le sujet pendant la période de non-affichage ; dans lequel
le processus de définition de région comporte en outre la définition de la région spécifiée,
le processus de calcul de données d'évaluation comporte en outre le calcul, sur la base des données de détermination, de données de temps de présence qui sont basées sur le temps de présence pendant lequel le point de regard (165) est présent dans la région correspondante (A1 à A5) pendant la période de non-affichage (T4, T6, T8, T10, T12), les données de temps de présence comportant des premières données de temps de présence qui indiquent un premier temps de présence pendant lequel le point de regard (165) est présent dans la région spécifiée pendant la période de non-affichage (T4, T6, T8, T10, T12), et des deuxièmes données de temps de présence qui indiquent un deuxième temps de présence pendant lequel le point de regard (165) est présent dans la région correspondante (A1 à A5) qui n'est pas la région spécifiée pendant la période de non-affichage (T4, T6, T8, T10, T12),
le processus d'exécution comporte l'exécution de l'opération d'affichage et de l'opération de non-affichage de façon répétitive à plusieurs reprises, et
le processus de calcul des données d'évaluation comporte le calcul des données d'évaluation sur la base des données de région et des données de temps de présence, dans lequel
le processus de calcul des données de région comporte le calcul des premières données de temps de présence et des deuxièmes données de temps de présence tout au long des périodes de non-affichage (T4, T6, T8, T10, T12) de l'opération de non-affichage, ou le calcul des premières données de temps de présence et des deuxièmes données de temps de présence pour chaque période de non-affichage (T4, T6, T8, T10, T12) de l'opération de non-affichage.
